(19) Europäisches Patentamt
European Patent Office
Office européen des brevets

(11) EP 4 166 139 A1

(12) EUROPEAN PATENT APPLICATION
published in accordance with Art. 153(4) EPC

(43) Date of publication:
19.04.2023 Bulletin 2023/16

(21) Application number: 21821111.8

(22) Date of filing: 10.06.2021

(51) International Patent Classification (IPC):
*A61K 31/454* (2006.01) *A61P 17/00* (2006.01)
*A61P 37/08* (2006.01) *C07D 401/14* (2006.01)

(52) Cooperative Patent Classification (CPC):
A61K 31/454; A61P 17/00; A61P 37/08;
C07D 401/14

(86) International application number:
PCT/JP2021/022048

(87) International publication number:
WO 2021/251452 (16.12.2021 Gazette 2021/50)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 10.06.2020 JP 2020101162

(71) Applicant: Mitsubishi Tanabe Pharma Corporation
Osaka-shi,
Osaka 541-8505 (JP)

(72) Inventors:
- SUZUKI, Tsuyoshi
Osaka-shi, Osaka 541-8505 (JP)
- KONDO, Masahiro
Osaka-shi, Osaka 541-8505 (JP)
- KAWANO, Yuko
Osaka-shi, Osaka 541-8505 (JP)
- MATSUMOTO, Atsuhiro
Osaka-shi, Osaka 541-8505 (JP)

(74) Representative: Hoffmann Eitle
Patent- und Rechtsanwälte PartmbB
Arabellastraße 30
81925 München (DE)

# (54) PREVENTIVE OR THERAPEUTIC AGENT FOR ACTINIC SKIN DISEASE

(57) A medicament for treating or preventing a condition that is treatable or preventable by promotion of melanin production, such as photodermatoses (excluding porphyria), hypopigmentary disease (excluding vitiligo vulgaris), adverse effects of phototherapy, or gray hair, said medicament compirising a compound represented by general formula [I], or a pharmaceutically acceptable salt or cocrystal thereof, as an active ingredient.

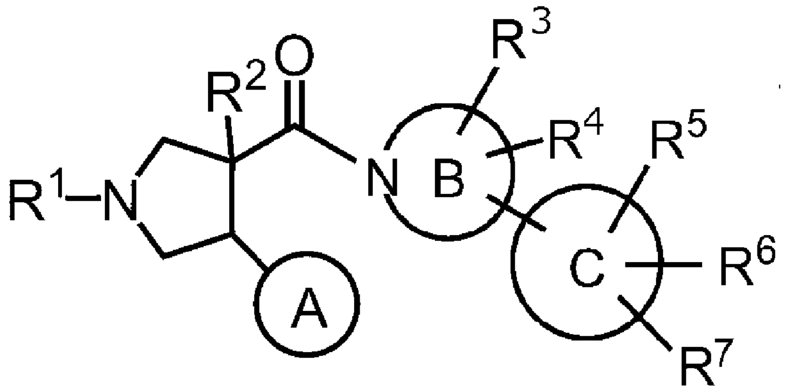

[I]

EP 4 166 139 A1

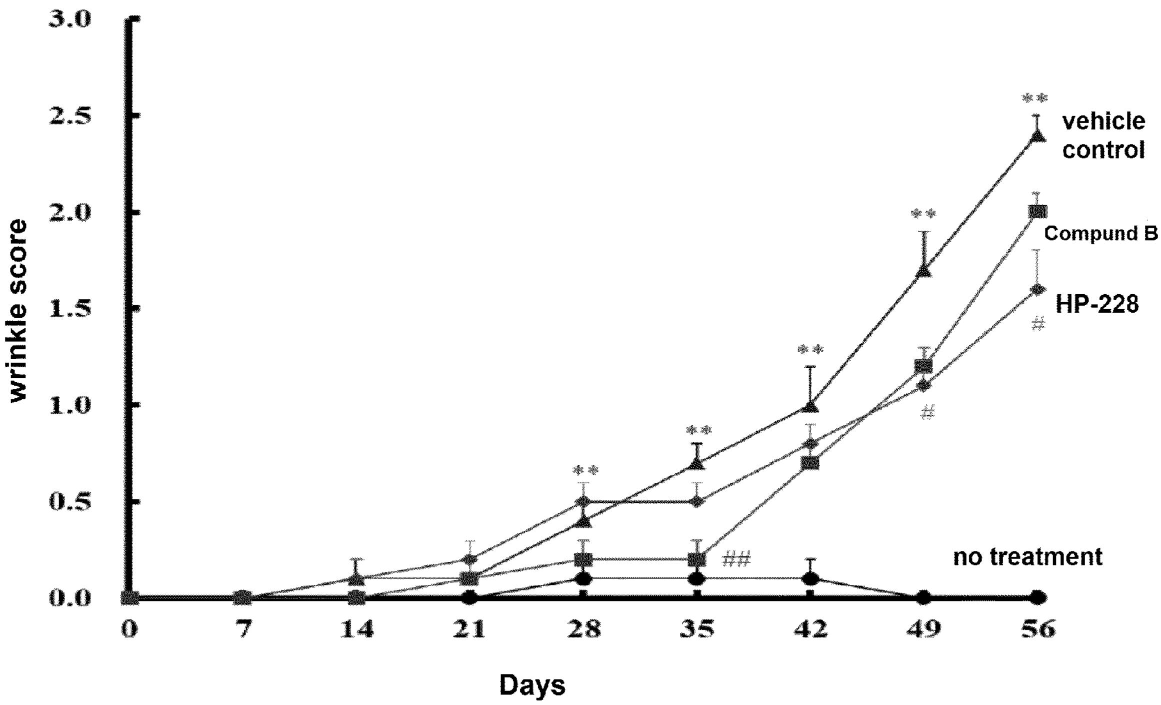
3.0
2.5
2.0
1.5
1.0
0.5
0.0
wrinkle score
vehicle
control
Compund B
HP-228
no treatment
**
#
##
0
7
14
21
28
35
42
49
56
Days

Fig. 3

## Description

### TECHNICAL FIELD

The present invention relates to a pharmaceutical composition for treatment or prevention of photodermatoses and the like by use of a compound having a melanocortin receptor (MCR) agonist activity.

### BACKGROUND ART

Since rays of light at a wavelength of 300 nm or less, among rays of light emitted from the sun, are absorbed by the stratospheric ozone layer, solar light reaching the ground corresponds to ultraviolet light, visible light, and infrared light at 300 nm or more. Examples of physiological responses occurring in catching rays of the sun include not only solar dermatitis (sunburn), but also any changes (wrinkle, sagging, pigmented patch) in the skin, called photoaging, which are caused by long-term exposure. On the other hand, any disease causing a pathological change such as dermatitis due to light irradiation at a level not causing any response in healthy subjects is called photodermatoses. Photodermatoses can be categorized into the following four classes: photoallergic dermatitis, DNA repair disorder, porphyria, and hydroa vacciniforme. The most primitive method as a method for treating photodermatoses is to block out light.

There is a phenomenon in which a brown pigment called melanin is deposited in the skin, as a physiological response in receiving of ultraviolet light irradiation by the human body. This is one of biological defense responses for protecting cells from DNA damage due to ultraviolet light. Cells producing melanin are called melanocytes, and precursor cells thereof are produced in the follicle and localized in the follicle, and simultaneously moved also to the basal epithelial layer of the skin. Melanocytes play important roles in decision of the hair color, the iris color, and the skin color of humans. A mechanism of melanin production by melanocytes involves binding $\alpha$-melanocyte-stimulating hormones ($\alpha$-MSHs) as in vivo ligands, to melanocortin receptors 1 (MC1Rs) on melanocyte cell surfaces, with solar light irradiation serving as environmental stress, to thereby activate intracellular signals and increase eumelanin synthesis.

Melanocortin receptors (MCRs) as receptors for $\alpha$-MSH are seven transmembrane G-protein-coupled receptors (GPCRs), and the activation thereof leads to a rise of intracellular cyclic AMP (cAMP), resulting in activation of downstream signals and expression of physiological activity. MCRs have five subtypes of MC1R to MC5R.

MC1R is a receptor which is mainly activated by $\alpha$-MSH, and is expressed in melanocytes, immune and inflammatory cells, fibroblasts, keratinocytes, endothelial cells, glial cells, and the like. Thus, the in vivo activation of MC1R is known to allow for the occurrence of various vital responses, and, for example, one phenomenon is known in which the cAMP level is increased in MC1R-expressing cells to result in various effects such as melanogenesis in the skin, maintenance of homeostasis against external stimuli, an anti-inflammatory action, and an action of inhibition of tissue fibrosis.

MC2R is a receptor which exhibits a low response against $\alpha$-MSH and is mainly activated by ACTH, and is highly expressed in the adrenal cortex. The activation of MC2R is known to allow for the exertion of a steroidogenesis effect.

MC3R is a receptor which is mainly activated by $\gamma$-MSH and ACTH and is expressed in central nerves, macrophage, and the like. The activation of MC3R is known to allow for the exertion of effects such as regulation of autonomic nervous function and an anti-inflammatory action.

MC4R is a receptor which is mainly activated by $\alpha$-MSH and ACTH and is expressed in central nerves and the like. Thus, the activation of MC4R is known to allow for the exertion of effects such as feeding suppression and an enhancement of erectile function.

MC5R is a receptor which is mainly activated by $\alpha$-MSH and is expressed in exocrine glands, lymphocytes, and the like. The activation of MC5R is known to exert effects such as exocrine fluid regulation and immune function regulation. Thus, the activation of such melanocortin receptors (MCRs) is expected to provide effects such as immune regulation, anti-inflammation, and suppression of tissue fibrosis through formation of cAMP.

Thus, not only MCR is thus classified to subtypes and such subtypes are different in function from one another, but also there are various actions downstream of signaling in each of such subtypes, and therefore it is important to specifically control a target receptor by a substance which induces an objective action, in order that a target pharmacological action is efficiently exerted with any adverse effects being suppressed.

For example, it has been considered to use a substance having an action of activating MC1R and thus increasing melanin production, in the treatment of photodermatoses, and there has been a need for a MC1R selective agonist to be used for the purpose of treatment of photodermatoses and the like (excluding protoporphyria and vitiligo vulgaris).

Patent Documents 1 to 6 each disclose afamelanotide which is, although not MC1R-selective, a substance for activation of MCR including MC1R and which can be used for the purposes of treatment of photodermatoses, a decrease in adverse effects of phototherapy, and the like. However, afamelanotide is not a MC1R selective agonist and thus leads to concerns about the incidence of adverse effects, and is a peptide and thus cannot be orally administered and has the

problems of, for example, a short half-life and a need for periodic subcutaneous implant by a healthcare professional.

Patent Documents 7 and 8 each disclose a pyrrolidine compound such as 1-{2-[(3S,4R)-1-{[(3R,4R)-1-cyclopentyl-3-fluoro-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid, or a pharmaceutically acceptable salt, hydrate/solvate or cocrystal, which has an excellent activation action of MCR, particularly MC1R. The Documents each disclose the compound which is useful for preventing or treating various diseases or symptoms associated with the activation of MCR, particularly MC1R, and specific examples of such diseases or symptoms include rheumatoid arthritis, gouty arthritis, osteoarthritis, inflammatory bowel disease, systemic sclerosis, psoriasis, fibrosis, protoporphyria, systemic lupus erythematosus, melanoma, skin cancer, vitiligo vulgaris, alopecia, pain, ischemia/reperfusion injury, cerebral inflammatory disease, hepatitis, sepsis/septic shock, nephritis, transplantation, HIV disease exacerbation, vasculitis, uveitis, retinitis pigmentosa, age-related macular degeneration, microbial psoriasis, celiac disease, nephrotic syndrome, or melanoma invasion. However, the compound is not disclosed to be effective for treating or preventing photodermatoses (excluding protoporphyria), hypopigmentary disease (excluding vitiligo vulgaris), adverse effects of phototherapy, gray hair, and the like, based on a mechanism mainly involving an increase of pigmentation.

PRIOR ART REFERENCES

PATENT DOCUMENTS

Patent Document 1: WO2014/160015
Patent Document 2: WO2018/142318
Patent Document 3: WOO2008/025094
Patent Document 4: WO2009/103816
Patent Document 5: WO2015/067503
Patent Document 6: WO2008/025094
Patent Document 7: WO2015/182723
Patent Document 8: WO2020/138481

SUMMARY OF THE INVENTION

Problems to be solved by the invention

There are few methods for fundamentally treating photodermatoses, and a method for preventing the skin from being exposed to sunlight is mainly adopted for avoiding any symptom from being developed or alleviating any symptom. In particular, a patient suffering from photodermatoses induced by visible light also tends to avoid outings in daylight, and quality of life (QOL) of the patient is known to be remarkably impaired.

While analogues of $\alpha$-melanocyte-stimulating hormones ($\alpha$-MSHs) as ligands of MCR have been developed as treating agents of photodermatoses and the like (Patent Documents 1 to 6), as described above, such analogues are not MC1R selective agonists, and lead to concerns about the incidence of adverse effects. Such analogues are peptides, thus cannot be orally administered and have a need for periodic subdermal implant by a healthcare worker, and therefore are problematic in terms of convenience.

Thus, there is a need for a pharmaceutical composition for treatment or prevention of photodermatoses and the like, which can allow for effective treatment in a safer manner without any load burden on patients.

Means to solve the problems

The present inventors have made intensive studies in order to solve the above problems. As a result, the present inventors have found that a compound represented by the following general formula [I], or a pharmaceutically acceptable salt or cocrystal thereof efficiently promotes melanin production in vivo to promote skin pigmentation, and thus is effective for preventing and/or treating photodermatoses (excluding protoporphyria), hypopigmentary disease (excluding vitiligo vulgaris), adverse effects of phototherapy, gray hair, and the like. The present inventors have further found a particularly effective amount of dosage for pigmentation in humans. The present inventors have led to completion of the present invention based on such findings.

The present invention provides a medicament for treating or preventing a condition (excluding protoporphyria and vitiligo vulgaris) that is treatable or preventable mainly by promotion of melanin production, the medicament comprising a compound represented by the following general formula [I], or a pharmaceutically acceptable salt or cocrystal

thereof, as an active ingredient.

The present invention provides a method for treating or preventing a condition (excluding protoporphyria and vitiligo vulgaris) that is treatable or preventable mainly by promotion of melanin production, the method comprising administering an effective amount of a compound represented by the following general formula [I], or a pharmaceutically acceptable salt or cocrystal thereof to a subject in need of such treating or preventing.

The present invention provides a compound represented by the following general formula [I], or a pharmaceutically acceptable salt or cocrystal thereof, for use in treating or preventing a condition (excluding protoporphyria and vitiligo vulgaris) that is treatable or preventable mainly by promotion of melanin production.

The present invention provides use of a compound represented by the following general formula [I], or a pharmaceutically acceptable salt or cocrystal thereof, in manufacture of a medicament for treating or preventing a condition (excluding protoporphyria and vitiligo vulgaris) that is treatable or preventable mainly by promotion of melanin production.

The compound represented by general formula [I] is here preferably 1-{2-[(3S,4R)-1-{[(3R,4R)-1-cyclopentyl-3-fluoro-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid, or a pharmaceutically acceptable salt or cocrystal thereof (hereinafter, also referred to as "compound A".).

Examples of the condition that is treatable or preventable mainly by promotion of melanin production include photodermatoses (excluding protoporphyria), hypopigmentary disease (excluding vitiligo vulgaris), adverse effects of phototherapy, and gray hair, and examples of such photodermatoses include photoallergic dermatitis, hydroa vacciniforme, DNA repair disorder, and porphyria (excluding protoporphyria). Examples of such photoallergic dermatitis include solar urticaria, polymorphous light eruption, chronic actinic dermatitis, photoallergic contact dermatitis, and photoallergic drug eruption, examples of such DNA repair disorder include xeroderma pigmentosum, Cockayne syndrome, Bloom syndrome, Werner syndrome, and Rothmund-Thomson syndrome, Trichothiodystrophy, and UV-sensitive syndrome, and examples of such porphyria (excluding protoporphyria) include congenital erythropoietic porphyria, variegate porphyria, acute intermittent porphyria, porphyria cutanea tarda and hereditary coproporphyria. In particular, preferable are, for example, photoallergic dermatitis such as solar urticarial and polymorphous light eruption, porphyria (excluding protoporphyria) such as congenital erythropoietic porphyria, variegate porphyria, acute intermittent porphyria, porphyria cutanea tarda and hereditary coproporphyria, and DNA repair disorders such as xeroderma pigmentosum. Examples of such hypopigmentary disease (excluding vitiligo vulgaris) include congenital hypopigmentary diseases such as oculocutaneous albinism, piebaldism, nevus depigmentosus, hypomelanosis of Ito, phenylketonuria, tuberous sclerosis, dyschromatosis symmetrica hereditaria, Waardenburg syndrome, Hermansky-Pudlak syndrome, Chediak-Higashi syndrome, Griscelli syndrome, and Tietz syndrome, and acquired hypopigmentary diseases such as leukoderma Sutton, Vogt-Koyanagi-Harada syndrome, leukoderma senile, leukoderma after sea bathing, and leukoderma syphiliticum.

The amount of dosage of the compound represented by general formula [I], or the pharmaceutically acceptable salt or cocrystal thereof, is preferably 50 to 500 mg/day, more preferably 100 to 300 mg/day.

## EFFECTS OF THE INVENTION

According to the present invention, it is possible to efficiently promote melanin production and thus promote skin pigmentation, without any need for catching of ultraviolet light as representative of rays of the sun, and effects by MC1R activation, mainly any actions, enable photodermatoses, hypopigmentary disease, adverse effects of phototherapy, gray hair, and the like to be treated and/or prevented. A compound represented by general formula [I] or a pharmaceutically acceptable salt or cocrystal thereof, which not only can be orally administered, but also is a MC1R-selective compound favorable in disposition in humans, has few adverse effects and can allow photodermatoses, hypopigmentary disease, adverse effects of phototherapy, gray hair, and the like to be safely treated and/or prevented effectively without any burdens on patients.

## BRIEF DESCRIPTION OF THE DRAWINGS

[Figure 1] Figure 1 is a diagram representing the results of a melanin production assay with Compound A or NDP-αMSH in mouse melanoma derived cell line B16F1 cells. EC50 value of Compound A and NDP- α MSH are 13.00 and 8.455, respectively. Each value represents the mean ± standard error of three experiments. * and ** indicate $P < 0.05$ and $P < 0.01$, respectively.

[Figure 2] Figure 2 is a diagram indicating the change in skin melanin density observed until day 57 in healthy subjects who received repeated administration of Compound A for 14 days.

[Figure 3] Figure 3 shows the changes in skin wrinkle scores over time in the UV-A irradiated mouse photoaging

model when compound B or HP-228 (reference control, melanocortin receptor-activating peptide, (Neuropeptides 1997, 31, 565)) was administered. Each value represents the mean ± standard error of 10 mice. ** indicates P<0.01 between the no-treatment group and the vehicle control group. ## and ### indicate P<0.05 and P<0.01, respectively, for the test substance-treated group compared with the vehicle control group.

[Figure 4] Figure 4 shows the amount of type I collagen in excised skin after 8 weeks of treatment with compound B or HP-228 (reference control, melanocortin receptor-activating peptide) in the UV-A-irradiated mouse photoaging model. Each value represents the mean ± standard error of 10 mice. ** indicates P<0.01 between the no-treatment group and the vehicle control group. ## indicates P<0.01 for the test substance-treated group compared with the vehicle control group.

EMBODIMENTS FOR CARRYING OUT THE INVENTION

Hereinafter, the present invention will be described.

<Definitions of substituents>

Definitions of groups herein can be freely combined, unless particularly specified.

The "alkyl" herein refers to a linear or branched saturated hydrocarbon chain group having 1 to 6 carbon atoms (C1 to 6). Especially, a group having 1 to 4 carbon atoms (C1 to 4) is preferable. Specific examples include methyl, ethyl, n-propyl, i-propyl, n-butyl, t-butyl, 2-methyl-n-butyl, i-amyl (3-methyl-n-butyl), and 2-methyl-n-pentyl. Especially, methyl, ethyl, i-propyl, or t-butyl is preferable.

The "alkenyl" refers to a linear or branched hydrocarbon chain group having at least one double bond and having 2 to 6 carbon atoms (C2 to 6). Examples especially include a group having 2 to 4 carbon atoms (C2 to 4). Specific examples include vinyl, propenyl, and butenyl.

The "cycloalkyl" refers to a monocyclic saturated hydrocarbon group having 3 to 7 carbon atoms (C3 to 7), and adamanthyl, and specific examples thereof include cyclopropyl, cyclobutyl, cyclopentyl, cyclohexyl, cycloheptyl, and adamanthyl.

The "cycloalkenyl" refers to a cyclic group having at least one double bond and having 3 to 7 carbon atoms (C3 to 7). Specific examples include cyclopropenyl, cyclobutenyl, cyclopentenyl, and cyclohexenyl.

The "alkoxy" refers to a monovalent group where the alkyl is bound to an oxygen atom, examples thereof include linear or branched alkyl-O- having 1 to 6 carbon atoms (C1 to 6), and alkyl-O- having 1 to 4 carbon atoms (C1 to 4) is preferable. Specific examples include methoxy, ethoxy, n-propoxy, i-propoxy, n-butoxy, t-butoxy, 2-methyl-n-propoxy, and 3-methyl-n-butoxy.

The "alkanoyl" refers to a group where carbonyl (C=O) is bound to the alkyl, examples thereof include linear or branched alkyl-C(=O)- having 1 to 6 carbon atoms (C1 to 6), and alkyl-C(=O)- having 1 to 4 carbon atoms (C1 to 4) is preferable. Specific examples include acetyl, propionyl, and butyryl.

The "alkylene" refers to a divalent linear or branched saturated hydrocarbon group having 1 to 6 carbon atoms (C1 to 6), and a group having 1 to 4 carbon atoms (C1 to 4) is preferable. Specific examples include methylene, ethylene, trimethylene (propylene), and tertamethylene (n-butylene).

The "alkyleneoxy" refers to a divalent group where an oxygen atom is bound to the alkylene, specific examples thereof include alkylene-O- having 1 to 6 carbon atoms (C1 to 6), and alkylene-O- having 1 to 4 carbon atoms (C1 to 4) is preferable. The alkyleneoxy group is optionally substituted concurrently on different two atoms (for example, carbon atoms), or is optionally substituted on the same atom (for example, carbon atom) to form a spiro ring.

Examples of halogen or halo include a fluorine atom, a chlorine atom, a bromine atom, or an iodine atom. Examples especially include a fluorine atom and a chlorine atom.

The "haloalkyl" refers to alkyl substituted with 1 to 3 halogen atoms, and specific examples thereof include difluoromethyl, trifluoromethyl, 1-fluoromethyl, and 2-fluoroethyl.

The "haloalkoxy" refers to alkyl-O- substituted with 1 to 3 halogen atoms, and specific examples thereof include trifluoromethoxy.

The "hydroxyalkyl" refers to alkyl substituted with one hydroxyl group, and specific examples thereof include hydroxymethyl, hydroxyethyl, 2-hydroxy-1,1-dimethylethyl, and 4-hydroxy-4-methyl-n-pentyl.

The "cyanoalkyl" refers to alkyl substituted with one cyano group, and specific examples thereof include cyanomethyl.

The "alkoxyalkyl" refers to alkyl substituted with one alkoxy group, and specific examples thereof include methoxymethyl, methoxyethyl, 2-methoxy-1,1-dimethylethyl, and 4-methoxy-4-methyl-n-pentyl.

Examples of aryl include a 6- to 10-membered aromatic hydrocarbon ring group. Monocyclic or bicyclic aryl is preferable, specific examples thereof include phenyl and naphthyl, and phenyl is especially preferable.

The "optionally partially hydrogenated aryl" encompasses both the aryl and the aryl partially hydrogenated, and

also encompasses, for example, a cyclic group formed by fusion of a phenyl group and a cycloalkyl group, and a cyclic group formed by fusion of a phenyl group and a cycloalkenyl group. Specific examples include phenyl, naphthyl, dihydrophenyl, indanyl, dihydronaphthyl, and tetrahydronaphthyl.

The "heteroaryl" refers to a 5- to 10-membered monocyclic or bicyclic group including 1 to 4 hetero atoms independently selected from the group consisting of a sulfur atom, an oxygen atom, and a nitrogen atom. Examples preferably include 5-to 6-membered monocyclic heteroaryl including at least one nitrogen atom and also optionally including a hetero atom independently selected from the group consisting of a sulfur atom, an oxygen atom, and a nitrogen atom. Another preferable heteroaryl is a 5- to 6-membered monocyclic heteroaryl group including 1 to 4 hetero atoms independently selected from the group consisting of an oxygen atom, a sulfur atom, and a nitrogen atom. Specific examples include pyrrolyl, furanyl, thienyl, imidazolyl, pyrazolyl, oxazolyl, isoxazolyl, thiazolyl, tetrazolyl, oxadiazolyl, pyridinyl, pyrazinyl, pyrimidinyl, pyridazinyl, thiazinyl, triazinyl, indolyl, isoindolyl, and benzimidazolyl.

The "aliphatic heterocyclic ring" refers to a 4- to 8-membered saturated cyclic group including 1 to 3 hetero atoms independently selected from the group consisting of an oxygen atom, a sulfur atom, and a nitrogen atom. The aliphatic heterocyclic ring may form a bicyclic group or a tricyclic group by crosslinking of two carbon atoms forming the ring, with an alkylene group, or may have a double bond in the ring. Preferably, the aliphatic heterocyclic ring is a 4- to 7-membered monocyclic aliphatic heterocyclic ring including at least one nitrogen atom and optionally further including one hetero atom selected from the group consisting of an oxygen atom, a sulfur atom, and a nitrogen atom. Another preferable example is a 5- to 6-membered monocyclic aliphatic heterocyclic ring including 1 to 2 hetero atoms independently selected from the group consisting of an oxygen atom, a sulfur atom, and a nitrogen atom. Specific examples include azetidinyl, pyrrolidinyl, tetrahydrofuranyl, imidazolinyl, thiazolidinyl, isothiazolidinyl, piperidinyl, piperazinyl, morpholinyl, thiomorpholinyl, tetrahydropyranyl, dihydropyridinyl, homopiperazinyl, homomorpholinyl, 3-azabicyclo[3.1.0]-hexyl, and octahydropyrrolo[3,4-c]pyrrolyl. For example, azetidinyl, pyrrolidinyl, piperidinyl, morpholinyl, thiomorpholinyl, piperazinyl, and 3-azabicyclo[3.1.0]-hexyl are preferable. For example, pyrrolidinyl, piperidinyl, piperazinyl, and morpholinyl are preferable, and pyrrolidinyl, piperidinyl, and morpholinyl are particularly preferable. In addition, for example, tetrahydrofuranyl, tetrahydropyranyl, and piperidinyl are also preferable.

The "aliphatic heterocyclic carbonyl" refers to a group where a carbonyl group is bound to the aliphatic heterocyclic ring, and is preferably 4- to 7-membered monocyclic aliphatic heterocyclic ring-C(=O)- including 1 to 3 hetero atoms independently selected from the group consisting of a sulfur atom, an oxygen atom, and a nitrogen atom. More preferable is 4- to 7-membered monocyclic aliphatic heterocyclic carbonyl including at least one nitrogen atom and optionally further including one hetero atom selected from the group consisting of a sulfur atom, an oxygen atom, and a nitrogen atom. In particular, preferable is 5- to 6-membered monocyclic aliphatic heterocyclic carbonyl including at least one nitrogen atom, where a carbonyl group is bound to a nitrogen atom in the ring.

The "aliphatic heterocyclic sulfonyl" refers to a group where a sulfonyl group is bound to the aliphatic heterocyclic ring, and examples thereof include 4- to 7-membered monocyclic aliphatic heterocyclic ring-($SO_2$)- including 1 to 3 hetero atoms independently selected from the group consisting of a sulfur atom, an oxygen atom, and a nitrogen atom. Especially, preferable is 4- to 7-membered monocyclic aliphatic heterocyclic ring-($SO_2$)- including at least one nitrogen atom and optionally further including one hetero atom selected from the group consisting of a sulfur atom, an oxygen atom, and a nitrogen atom. In particular, preferable is 5- to 6-membered monocyclic aliphatic heterocyclic ring-($SO_2$)- including at least one nitrogen atom, where a sulfonyl group is bound to a nitrogen atom.

<Compound represented by general formula [I]>

Each symbol in the general formula [I] will be described below.

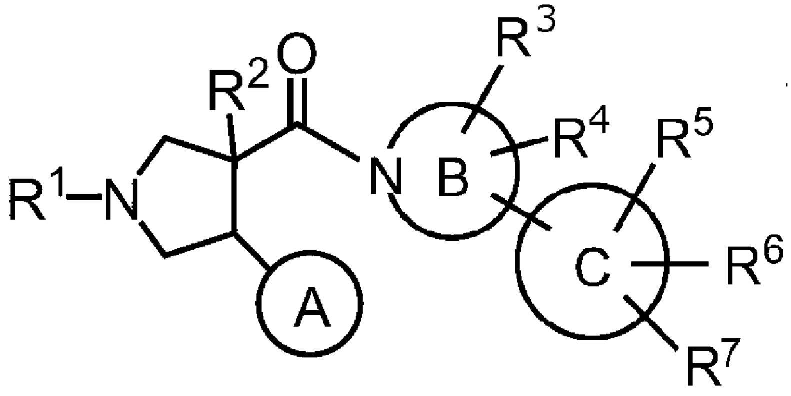

[I]

In the formula, ring A represents an optionally substituted aryl group or an optionally substituted heteroaryl group,

$R^1$ represents an optionally substituted alkyl group, an optionally substituted cycloalkyl group, an optionally substituted aliphatic heterocyclic group, an optionally substituted and optionally partially hydrogenated aryl group, an optionally substituted heteroaryl group, or a carbamoyl group optionally substituted with 1 to 2 alkyl groups,
$R^2$ represents a halogen atom, an alkyl group, or an alkoxy group;
ring B represents a nitrogen-containing aliphatic heterocyclic group optionally partially including a double bond,
ring C represents an aryl group or a heteroaryl group,
$R^3$ and $R^4$ each independently represent a group selected from the group consisting of a hydrogen atom, a halogen atom, a cyano group, an alkyl group, a haloalkyl group, a cyanoalkyl group, a hydroxyalkyl group, an alkoxyalkyl group, a carboxyl group, a carbamoyl group optionally substituted with 1 to 2 alkyl groups, and an alkoxy group;
$R^5$ represents an optionally substituted alkyl group, an optionally substituted alkenyl group, an optionally substituted cycloalkyl group, an optionally substituted cycloalkenyl group, an optionally substituted aryl group, an optionally substituted heteroaryl group, an optionally substituted aliphatic heterocyclic group, an optionally substituted alkoxy group, an amino group optionally substituted with 1 to 2 alkyl groups optionally substituted with a carboxyl group, or a carbamoyl group optionally substituted with 1 to 2 alkyl groups optionally substituted with a carboxyl group, and
$R^6$ and $R^7$ each independently represent a group selected from the group consisting of a hydrogen atom, a halogen atom, a cyano group, an alkyl group, a haloalkyl group, and a haloalkoxy group.

Examples of the aryl moiety of the "optionally substituted aryl group" represented by ring A include 6- to 10-membered monocyclic or bicyclic aryl, and specific examples thereof include phenyl or naphthyl. Especially, a phenyl group is preferable.

The heteroaryl moiety of the "optionally substituted heteroaryl group" represented by ring A is preferably 5- to 6-membered monocyclic heteroaryl including at least one nitrogen atom and optionally further 1 to 3 hetero atoms independently selected from the group consisting of an oxygen atom, a sulfur atom, and a nitrogen atom. Specific examples include a pyrrolyl group, a furanyl group, thienyl group, a pyridinyl group, a pyrimidinyl group, and a pyridazinyl group. A pyridinyl group is particularly preferable.

The substituents of the "optionally substituted aryl group" and the "optionally substituted heteroaryl group" each represented by ring A may each correspond to 1 to 3 groups independently selected, and examples thereof include a halogen atom, an alkyl group, a haloalkyl group, a cycloalkyl group, an alkoxy group, a haloalkoxy group, and an alkyleneoxy group. Specific examples include a fluorine atom, a chlorine atom, a methyl group, an ethyl group, an i-propyl group, a trifluoromethyl group, a cyclopropyl group, a methoxy group, an ethoxy group, a trifluoromethoxy group, and an ethyleneoxy group.

Examples of ring A include an aryl group optionally substituted with 1 to 2 groups independently selected from the group consisting of a halogen atom, an alkyl group, a haloalkyl group, a cycloalkyl group, an alkoxy group, a haloalkoxy group, and an alkyleneoxy group; and a heteroaryl group optionally substituted with 1 to 2 groups independently selected from the group consisting of a halogen atom and an alkoxy group. Preferable are an aryl group optionally substituted with 1 to 2 groups independently selected from the group consisting of a halogen atom, an alkyl group, a haloalkyl group, a cycloalkyl group, an alkoxy group, a haloalkoxy group, and an alkyleneoxy group, and a heteroaryl group optionally substituted with a halogen atom or an alkoxy group. More preferable is an aryl group optionally substituted with 1 to 2

groups independently selected from the group consisting of a halogen atom, an alkyl group, a haloalkyl group, a cycloalkyl group, an alkoxy group, a haloalkoxy group, and an alkyleneoxy group, and particularly preferable is an aryl group optionally substituted with an alkoxy group.

Examples of the "phenyl substituted with an alkyleneoxy group" in the "aryl group substituted with an alkyleneoxy group" include the following structures.

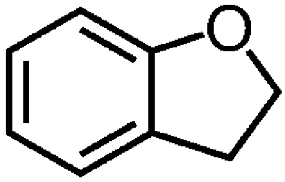 , 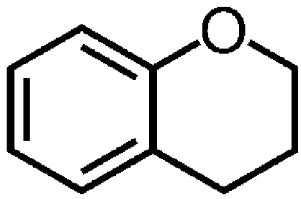 , 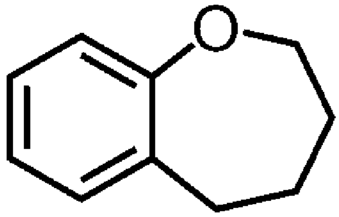

Examples of ring A also include an aryl group optionally substituted with 1 to 2 groups independently selected from the group consisting of a halogen atom, an alkyl group, a haloalkyl group, a cycloalkyl group, an alkoxy group, a haloalkoxy group, and an alkyleneoxy group; and a heteroaryl group optionally substituted with 1 to 2 groups independently selected from the group consisting of a halogen atom and an alkoxy group, more specifically include a phenyl group optionally substituted with 1 to 2 groups independently selected from the group consisting of a fluorine atom, a chlorine atom, a methyl group, an ethyl group, an i-propyl group, a trifluoromethyl group, a cyclopropyl group, a methoxy group, an ethoxy group, and a trifluoromethoxy group; a dihydrobenzofuranyl group; a pyridinyl group optionally substituted with a fluorine atom; and a pyridinyl group optionally substituted with a methoxy group. Preferable is a phenyl group optionally substituted with 1 to 2 groups independently selected from the group consisting of a fluorine atom, a chlorine atom, a methyl group, an ethyl group, an i-propyl group, a trifluoromethyl group, a cyclopropyl group, a methoxy group, an ethoxy group, and a trifluoromethoxy group, and more preferable is a phenyl group optionally substituted with a methoxy group.

The alkyl of the "optionally substituted alkyl group" represented by $R^1$ is especially preferably t-butyl.

The cycloalkyl of the "optionally substituted cycloalkyl group" represented by $R^1$ is especially preferably cyclopentyl or cyclohexyl.

The aliphatic heterocyclic ring of the "optionally substituted aliphatic heterocyclic group" represented by $R^1$ is preferably a 5- to 6-membered monocyclic aliphatic heterocyclic group having 1 to 2 hetero atoms independently selected from the group consisting of an oxygen atom, a sulfur atom, and a nitrogen atom. Specifically, tetrahydrofuranyl, tetrahydropyranyl, or piperidinyl is preferable.

Examples of the optionally partially hydrogenated aryl of the "optionally substituted and optionally partially hydrogenated aryl group" represented by $R^1$ include phenyl, naphthyl, and indanyl. Especially, indanyl is preferable.

The heteroaryl of the "optionally substituted heteroaryl group" represented by $R^1$ is preferably a 5- to 6-membered monocyclic heteroaryl group including 1 to 4 hetero atoms independently selected from the group consisting of an oxygen atom, a sulfur atom, and a nitrogen atom. Especially, preferable is 5- to 6-membered monocyclic nitrogen-containing heteroaryl including at least one nitrogen atom and optionally further including one hetero atom selected from the group consisting of an oxygen atom, a sulfur atom, and a nitrogen atom. Specific examples include pyrrolyl, imidazolyl, pyrazolyl, oxazolyl, isoxazolyl, thiazolyl, oxadiazolyl, pyridinyl, pyrazinyl, pyrimidinyl, pyridazinyl, thiazinyl, and triazinyl. Examples particularly preferably include pyridinyl, pyridazinyl, and pyrimidinyl.

The substituents of the "optionally substituted alkyl group", the "optionally substituted cycloalkyl group", the "optionally substituted aliphatic heterocyclic group", the "optionally substituted and optionally partially hydrogenated aryl group", the "optionally substituted heteroaryl group", and the "optionally substituted carbamoyl group" each represented by $R^1$ may each correspond to 1 to 3 groups, preferably 1 to 2 groups, which are the same or different, and examples thereof include a halogen atom; a hydroxyl group; an oxo group; a cyano group; an alkyl group; a hydroxyalkyl group; an alkoxyalkyl group; a haloalkyl group; a cycloalkyl group; an alkoxy group; an alkanoyl group; an alkylsulfonyl group; an aliphatic heterocyclic group; an aliphatic heterocyclic carbonyl group optionally substituted with 1 to 2 groups independently selected from the group consisting of a halogen atom, an alkyl group, a haloalkyl group, and an alkoxyalkyl group; an aliphatic heterocyclic sulfonyl group; a carbamoyl group optionally substituted with 1 to 2 alkyl groups; and an alkyleneoxy group.

More particularly, examples include a halogen atom; a hydroxyl group; an oxo group; a cyano group; an alkyl group; a hydroxyalkyl group; an alkoxyalkyl group; a haloalkyl group; a cycloalkyl group; an alkoxy group; an alkanoyl group; an alkylsulfonyl group; an aliphatic heterocyclic group (wherein the aliphatic heterocyclic group is a 4- to 7-membered monocyclic aliphatic heterocyclic group including 1 to 2 hetero atoms independently selected from the group consisting of an oxygen atom, a sulfur atom, and a nitrogen atom); an aliphatic heterocyclic carbonyl group optionally substituted with 1 to 2 groups independently selected from the group consisting of a halogen atom, an alkyl group, a

haloalkyl group, and an alkoxyalkyl group (wherein the aliphatic heterocyclic ring is a 4- to 7-membered monocyclic aliphatic heterocyclic ring including at least one nitrogen atom and optionally further including one hetero atom selected from the group consisting of an oxygen atom, a sulfur atom, and a nitrogen atom); an aliphatic heterocyclic sulfonyl group (wherein the aliphatic heterocyclic ring is a 4- to 7-membered monocyclic aliphatic heterocyclic ring including at least one nitrogen atom and optionally further including one hetero atom selected from the group consisting of an oxygen atom, a sulfur atom, and a nitrogen atom); a carbamoyl group optionally substituted with 1 to 2 alkyl groups; and an alkyleneoxy group.

The "cycloalkyl group substituted with an alkyleneoxy group" herein encompasses any group (namely, spiro ring) where an alkyleneoxy group is bound to any identical carbon atom on the cycloalkyl group, and, for example, a cyclohexyl group substituted with a trimethyleneoxy group (propyleneoxy group) encompasses the following group.

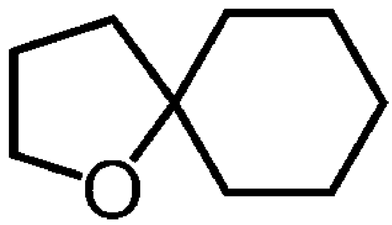

The substituent of the "optionally substituted alkyl group" represented by $R^1$ may correspond to 1 to 3 groups, preferably 1 to 2 groups, which are the same or different, and examples thereof include a halogen atom; a hydroxyl group; a cycloalkyl group; an alkoxy group; an aliphatic heterocyclic group; an aliphatic heterocyclic carbonyl group optionally substituted with 1 to 2 groups independently selected from the group consisting of a halogen atom, an alkyl group, a haloalkyl group, and an alkoxyalkyl group; an aliphatic heterocyclic sulfonyl group; and a carbamoyl group optionally substituted with 1 to 2 alkyl groups.

More particularly, examples include 1 to 2 groups independently selected from the group consisting of a halogen atom; a hydroxyl group; a cycloalkyl group; an alkoxy group; an aliphatic heterocyclic group (wherein the aliphatic heterocyclic group is a 4- to 7-membered monocyclic aliphatic heterocyclic group including at least one oxygen atom and optionally further including one hetero atom selected from the group consisting of an oxygen atom, a sulfur atom, and a nitrogen atom); an aliphatic heterocyclic carbonyl group optionally substituted with 1 to 2 groups independently selected from the group consisting of a halogen atom, an alkyl group, a haloalkyl group, and an alkoxyalkyl group (wherein the aliphatic heterocyclic ring is a 4- to 7-membered monocyclic aliphatic heterocyclic ring including at least one nitrogen atom and optionally further including one hetero atom selected from the group consisting of an oxygen atom, a sulfur atom, and a nitrogen atom); an aliphatic heterocyclic sulfonyl group (wherein the aliphatic heterocyclic ring is a 4- to 7-membered monocyclic aliphatic heterocyclic ring including at least one nitrogen atom and optionally further including one hetero atom selected from the group consisting of an oxygen atom, a sulfur atom, and a nitrogen atom); and a carbamoyl group optionally substituted with 1 to 2 alkyl groups.

More specifically, examples include a fluorine atom; a hydroxyl group; a cyclopropyl group; a cyclobutyl group; a methoxy group; a tetrahydropyranyl group; a pyrrolidinylcarbonyl group optionally substituted with a group selected from the group consisting of a fluorine atom, a methyl group, a trifluoromethyl group, and a methoxymethyl group; a piperidinylcarbonyl group optionally substituted with 1 to 2 fluorine atoms; a morpholinylcarbonyl group; a pyrrolidinylsulfonyl group; and a dimethylcarbamoyl group.

Examples of the "optionally substituted alkyl group" represented by $R^1$ include a methyl group, an ethyl group, an i-propyl group, an i-amyl group (3-methyl-n-butyl group), a 3-methyl-n-butyl group, a 1-fluoromethyl-2-fluoroethyl group, a t-butyl group, a cyclopropylmethyl group, a cyclobutylmethyl group, a 2-hydroxyethyl group, a 1,1-dimethyl-2-N,N-dimethylcarbamoyl-ethyl group, a 4-hydroxy-4-methyl-n-pentyl group, a 1,1-dimethyl-2-methoxyethyl group, a 4-methoxy-4-methyl-n-pentyl group, a N,N-dimethylaminocarbonylmethyl group, a 2-N,N-dimethylaminocarbonylethyl group, a 1,1-dimethyl-2-N,N-dimethylaminocarbonyl-ethyl group, a 3-N,N-dimethylaminocarbonyl-n-propyl group, a tetrahydropyranylmethyl group, a piperazinylcarbonylmethyl group, a pyrrolidinylcarbonyl group, a 2-methyl-pyrrolidinylcarbonylmethyl group, a 2-trifluoromethyl-pyrrolidinylcarbonylmethyl group, a 2-methoxymethyl-pyrrolidinylcarbonyl group, a 3-fluoro-pyrrolidinylcarbonylmethyl group, a morpholinylcarbonylmethyl group, a 4,4-difluoro-piperidinylcarbonylmethyl group, and a 3-pyrrolidinylsulfonylpropyl group, and a t-butyl group is especially preferable.

The substituent of the "optionally substituted cycloalkyl group" represented by $R^1$ may correspond to 1 to 3 groups which are the same or different, and examples thereof include a halogen atom, a hydroxyl group, an oxo group, a cyano group, an alkyl group, an alkoxy group, an alkoxyalkyl group, and an alkyleneoxy group. The alkyleneoxy group may be herein a substituent on the same carbon atom on cycloalkyl.

Examples of the substituent more particularly include 1 to 2 groups independently selected from the group

consisting of a halogen atom, a hydroxyl group, an oxo group, a cyano group, an alkyl group, an alkoxy group, an alkoxyalkyl group, and an alkyleneoxy group. More specifically, examples include 1 to 2 groups independently selected from the group consisting of a fluorine atom, a hydroxyl group, an oxo group, a cyano group, a methyl group, a methoxy group, an ethoxy group, an isopropoxy group, a methoxymethyl group, and a trimethyleneoxy group (propyleneoxy group). Especially, a methoxy group, an ethoxy group, and a cyano group are preferable.

Examples of the "optionally substituted cycloalkyl group" represented by $R^1$ include a cycloalkyl group optionally substituted with 1 to 2 groups independently selected from the group consisting of a halogen atom, a hydroxyl group, an oxo group, a cyano group, an alkyl group, an alkoxy group, an alkoxyalkyl group, and an alkyleneoxy group.

More particularly, examples include a 3- to 7-membered monocyclic cycloalkyl group optionally substituted with 1 to 3 groups independently selected from the group consisting of a halogen atom, a hydroxyl group, an oxo group, a cyano group, an alkyl group, an alkoxy group, an alkoxyalkyl group, and an alkyleneoxy group, and an adamanthyl group optionally substituted with a hydroxyl group.

More specifically, examples include a cyclopropyl group; a cyclobutyl group; a cyclopentyl group optionally substituted with 1 to 3 groups independently selected from the group consisting of a fluorine atom, a hydroxyl group, an oxo group, a cyano group, a methyl group, a methoxy group, an ethoxy group, an i-propoxy group, and a methoxymethyl group; a cyclohexyl group optionally substituted with 1 to 3 groups independently selected from the group consisting of a fluorine atom, a hydroxyl group, an oxo group, a cyano group, a methyl group, a methoxy group, an ethoxy group, an i-propoxy group, and a trimethyleneoxy group (propyleneoxy group); and a cycloheptyl group.

Examples especially preferably include a cyclopentyl group optionally substituted with one group selected from the group consisting of a methoxy group, an ethoxy group, and a cyano group; and a cyclohexyl group optionally substituted with one group selected from the group consisting of a methoxy group, an ethoxy group, and a cyano group.

Examples of the substituent of the "optionally substituted aliphatic heterocyclic group" represented by $R^1$ include an alkyl group, a hydroxyalkyl group, a haloalkyl group, an alkanoyl group, and an alkylsulfonyl group. More specifically, examples include a methyl group, a hydroxymethyl group, a 2-fluoro-1-fluoromethyl-ethyl group, an acetyl group, an ethylcarbonyl group, and an ethylsulfonyl group.

Examples of the "optionally substituted aliphatic heterocyclic group" represented by $R^1$ include a tetrahydrofuranyl group optionally substituted with an alkyl group, a hydroxy group, or a hydroxyalkyl group; a tetrahydropyranyl group optionally substituted with a hydroxyl group, an alkyl group, or a hydroxyalkyl group; and a piperidinyl group optionally substituted with a group selected from the group consisting of a haloalkyl group, an alkanoyl group, and an alkylsulfonyl group.

More specifically, examples include a tetrahydrofuranyl group; a tetrahydropyranyl group optionally substituted with a methyl group or a hydroxymethyl group; and a piperidinyl group optionally substituted with group selected from the group consisting of a 1-fluoromethyl-2-fluoroethyl group, an acetyl group, an ethylcarbonyl group, and an ethylsulfonyl group, and a tetrahydropyranyl group is preferable.

The "optionally substituted and optionally partially hydrogenated aryl group" represented by $R^1$ is preferably an indanyl group (especially, a 1-indanyl group, a 2-indanyl group, or the like).

The substituent of the "optionally substituted heteroaryl group" represented by $R^1$ may correspond to 1 to 3 groups which are the same or different, and examples thereof include a cyano group, an alkyl group, an alkoxy group, and a carbamoyl group. More specifically, examples include a cyano group, a methyl group, a methoxy group, and a carbamoyl group. Especially, a methyl group is preferable.

Examples of the "optionally substituted heteroaryl group" represented by $R^1$ include a pyridazinyl group optionally substituted with one group selected from the group consisting of a cyano group, an alkyl group, an alkoxy group, and a carbamoyl group; a pyridinyl group optionally substituted with 1 to 2 groups independently selected from the group consisting of a cyano group, an alkyl group, and an alkoxy group; and a pyrimidinyl group optionally substituted with an alkyl group.

More specifically, examples include a pyridazinyl group optionally substituted with one group selected from the group consisting of a cyano group, a methyl group, a methoxy group, and a carbamoyl group; a pyridinyl group optionally substituted with 1 to 2 groups independently selected from the group consisting of a cyano group, a methyl group, and a methoxy group; and a pyrimidinyl group optionally substituted with a methyl group, and, especially, a pyrimidinyl group optionally substituted with a methyl group and a pyridinyl group substituted with a methyl group are preferable.

Examples of the substituent of the "optionally substituted carbamoyl group" represented by $R^1$ include an alkyl group. More specifically, examples include a methyl group, an ethyl group, and an i-propyl group, and a methyl group is especially preferable.

Examples of the "optionally substituted carbamoyl group" represented by $R^1$ include a carbamoyl group, a monomethylcarbamoyl group, and an adimethylcarbamoyl group, and a carbamoyl group and a monomethylcarbamoyl group are preferable.

$R^1$ is preferably an alkyl group, an optionally substituted cycloalkyl group, an aliphatic heterocyclic group, or an optionally substituted heteroaryl group. More particularly, an alkyl group; a cycloalkyl group optionally substituted

with a cyano group or an alkoxy group; a 5- to 6-membered monocyclic aliphatic heterocyclic group including 1 to 2 hetero atoms independently selected from the group consisting of an oxygen atom, a sulfur atom, and a nitrogen atom; or a heteroaryl group optionally substituted with an alkyl group (wherein the heteroaryl group is a 5- to 6-membered monocyclic heteroaryl group including 1 to 4 hetero atoms independently selected from the group consisting of an oxygen atom, a sulfur atom, and a nitrogen atom). Specifically, a t-butyl group; a cyclopentyl group; a cyclohexyl group optionally substituted with a methoxy group, an ethoxy group, or a cyano group; a tetrahydropyranyl group; or a pyridinyl group optionally substituted with a methyl group is preferable.

$R^1$ is preferably an optionally substituted cycloalkyl group or an optionally substituted aliphatic heterocyclic group. More particularly, preferable is a 3- to 7-membered monocyclic cycloalkyl group optionally substituted with a cyano group or an alkoxy group; or an aliphatic heterocyclic group optionally substituted with an alkyl group, a haloalkyl group, or a hydroxyalkyl group (wherein the aliphatic heterocyclic group is a group selected from the group consisting of a tetrahydrofuranyl group, a tetrahydropyranyl group, a pyrrolidinyl group, and a piperidinyl group). Specifically, preferable is a cyclopentyl group; a cyclohexyl group optionally substituted with a methoxy group, an ethoxy group, or a cyano group; a piperidinyl group optionally substituted with a haloalkyl group; or a tetrahydropyranyl group optionally substituted with an alkyl group or a hydroxyalkyl group.

$R^1$ is particularly preferably an optionally substituted cycloalkyl group. More particularly, a cycloalkyl group optionally substituted with a cyano group or an alkoxy group is preferable. Specifically, a cyclopentyl group; or a cyclohexyl group optionally substituted with a group selected from the group consisting of a methoxy group, an ethoxy group, and a cyano group is preferable.

The alkyl of the "alkyl group" represented by $R^2$ is preferably C1 to 3 alkyl, specifically, for example, methyl, ethyl, or i-propyl is preferable.

The alkoxy of the "alkoxy group" represented by $R^2$ is preferably C 1 to 4 alkoxy, and is specifically, for example, methoxy, ethoxy, i-propoxy, or n-butoxy.

Suitable examples of $R^2$ include a halogen atom, an alkyl group, and an alkoxy group, and a halogen atom and an alkoxy group are more preferable. A fluorine atom or a methoxy group is especially preferable.

The "nitrogen-containing aliphatic heterocyclic group optionally partially including a double bond" represented by ring B is preferably a 4- to 8-membered monocyclic or bicyclic aliphatic heterocyclic ring including a nitrogen atom and optionally further including an oxygen atom or a sulfur atom. Specific examples include an azetidinyl group, a pyrrolidinyl group, an imidazolinyl group, a thiazolidinyl group, an isothiazolidinyl group, a piperidinyl group, a piperazinyl group, a morpholinyl group, a thiomorpholinyl group, a tetrahydropyridinyl group, a homopiperazinyl group, a homomorpholinyl group, a 3-azabicyclo[3.1.0]-hexyl group, or an octahydropyrrolo[3,4-c]pyrrolyl group. Examples preferably include an azetidinyl group, a pyrrolidinyl group, a piperidinyl group, a tetrahydropyridinyl group, a piperazinyl group, a homopiperazinyl group, and an octahydropyrrolo[3,4-c]pyrrolyl group. Still suitable examples include a pyrrolidinyl group and a piperidinyl group, and pyrrolidinyl is especially preferable.

Examples of the "aryl group" represented by ring C include monocyclic or bicyclic aryl, specific examples thereof include phenyl or naphthyl, and phenyl is preferable.

Examples of the "heteroaryl group" represented by ring C include 5- to 6-membered monocyclic heteroaryl including 1 to 4 hetero atoms independently selected from the group consisting of an oxygen atom, a sulfur atom, and a nitrogen atom. More specifically, pyridinyl is preferable.

Examples of $R^3$ and $R^4$ include a hydrogen atom, a halogen atom, a cyano group, an alkyl group, a haloalkyl group, a cyanoalkyl group, a hydroxyalkyl group, an alkoxyalkyl group, a carboxyl group, a carbamoyl group optionally substituted with 1 to 2 alkyl groups, and a group independently selected from the group consisting of an alkoxy group. More specifically, examples include a group independently selected from the group consisting of a hydrogen atom, a fluorine atom, a cyano group, a methyl group, a fluoromethyl group, a cyanomethyl group, a hydroxymethyl group, a 1-hydroxy-1-methylethyl group, a methoxymethyl group, an ethoxymethyl group, a carboxyl group, a dimethylcarbamoyl group, and a methoxy group.

Suitable examples include an example where $R^3$ is a group selected from the group consisting of a halogen atom, a cyano group, an alkyl group, a haloalkyl group, a cyanoalkyl group, a hydroxyalkyl group, an alkoxyalkyl group, a carboxyl group, a carbamoyl group optionally substituted with 1 to 2 alkyl groups, and an alkoxy group, and $R^4$ is a hydrogen atom; an example where $R^3$ is an alkoxyalkyl group and $R^4$ is a halogen atom, an alkyl group, or an alkoxy group; an example where both $R^3$ and $R^4$ are each a halogen atom; an example where both $R^3$ and $R^4$ are each an alkyl group; and an example where both $R^3$ and $R^4$ are each a hydrogen atom. More specifically, examples include an example where $R^3$ is a group selected from the group consisting of a fluorine atom, a cyano group, a methyl group, a fluoromethyl group, a cyanomethyl group, a hydroxymethyl group, a 1-hydroxy-1-methylethyl group, a methoxymethyl group, an ethoxymethyl group, a carboxyl group, a dimethylcarbamoyl group, and a methoxy group, and $R^4$ is a hydrogen atom; an example where $R^3$ is a methoxymethyl group and $R^4$ is a fluorine atom, a methyl group, or a methoxy group; an example where both $R^3$ and $R^4$ are each a fluorine atom; an example where both $R^3$ and $R^4$ are each a methyl group; and an example where both $R^3$ and $R^4$ are each a hydrogen atom.

The alkyl of the "optionally substituted alkyl group" represented by $R^5$ is preferably, for example, ethyl or n-butyl.

The alkenyl of the "optionally substituted alkenyl group" represented by $R^5$ is preferably, for example, n-butenyl.

The cycloalkyl of the "optionally substituted cycloalkyl group" represented by $R^5$ is preferably cyclohexyl.

The cycloalkenyl of the "optionally substituted cycloalkenyl group" represented by $R^5$ is especially preferably cyclohexenyl.

Examples of the aryl moiety of the "optionally substituted aryl group" represented by $R^5$ include phenyl and naphthyl, and phenyl is especially preferable.

The heteroaryl moiety of the "optionally substituted heteroaryl group" represented by $R^5$ is preferably 5- to 6-membered monocyclic heteroaryl including at least one nitrogen atom and optionally further including one hetero atom selected from an oxygen atom, a sulfur atom, or a nitrogen atom. Specifically, preferable are pyrrolyl, imidazolyl, pyrazolyl, oxazolyl, isoxazolyl, thiazolyl, tetrazolyl, oxadiazolyl, pyridinyl, pyrazinyl, pyrimidinyl, pyridazinyl, thiazinyl, and triazinyl. Especially, pyrazolyl and oxazolyl are preferable.

The aliphatic heterocyclic ring of the "optionally substituted aliphatic heterocyclic group" represented by $R^5$ is preferably a 4- to 8-membered monocyclic or bicyclic aliphatic heterocyclic ring including at least one nitrogen atom and optionally further including one hetero atom selected from the group consisting of an oxygen atom, a sulfur atom, and a nitrogen atom. Specifically, examples include azetidinyl, pyrrolidinyl, piperidinyl, piperazinyl, morpholinyl, thiomorpholinyl, and 3-azabicyclo[3.1.0]hexyl groups. Particularly suitable examples include piperidinyl.

The alkoxy of the "optionally substituted alkoxy group" represented by $R^5$ is preferably, for example, methoxy, n-propoxy, 2-methyl-n-propoxy, or 3-methyl-n-butoxy.

Specific examples of the "amino group optionally substituted with 1 to 2 alkyl groups optionally substituted with a carboxyl group" represented by $R^5$ include a N-methyl-N-3-carboxyl-n-propylamino group.

Examples of the "carbamoyl group optionally substituted with 1 to 2 alkyl groups optionally substituted with a carboxyl group" represented by $R^5$ preferably include a carbamoyl group.

The substituents of the "optionally substituted alkyl group", the "optionally substituted alkenyl group", the "optionally substituted cycloalkyl group", the "optionally substituted cycloalkenyl group", the "optionally substituted aryl group", the "optionally substituted heteroaryl group", the "optionally substituted aliphatic heterocyclic group", and the "optionally substituted alkoxy group" each represented by $R^5$ may each correspond to 1 to 2 groups which are the same or different, and examples thereof include 1 to 2 groups independently selected from the group consisting of a hydroxyl group; an oxo group; a cyano group; an alkyl group optionally substituted with a carboxyl group; an alkoxy group; an alkanoyl group; a carboxyl group; an alkoxycarbonyl group; an aliphatic heterocyclic carbonyl group optionally substituted with a carboxyl group (wherein the aliphatic heterocyclic ring is preferably a 4- to 7-membered monocyclic aliphatic heterocyclic ring, more preferably a 5- to 6-membered monocyclic aliphatic heterocyclic ring, including at least one nitrogen atom and optionally further including one hetero atom selected from the group consisting of an oxygen atom, a sulfur atom, and a nitrogen atom); a heteroaryl group optionally substituted with a hydroxyl group or a oxo group (wherein the heteroaryl group is preferably a 5- to 6-membered monocyclic heteroaryl group including 1 to 4 hetero atoms independently selected from the group consisting of an oxygen atom, a sulfur atom, and a nitrogen atom); an aliphatic heterocyclic group optionally substituted with 1 to 2 oxo groups (wherein the aliphatic heterocyclic group is preferably a 4- to 7-membered monocyclic aliphatic heterocyclic group, more preferably 5- to 6-membered monocyclic aliphatic heterocyclic group, including 1 to 2 hetero atoms independently selected from the group consisting of an oxygen atom, a sulfur atom, and a nitrogen atom); a carbamoyl group optionally substituted with 1 to 2 groups independently selected from the group consisting of an alkyl group and a hydroxyl group (wherein the alkyl is optionally substituted with a hydroxyl group, an alkoxy group, or a carboxyl group); an alkylsulfonyl group; an aminosulfonyl group optionally substituted with 1 to 2 alkyl groups; an alkylsulfonylaminocarbonyl group; an aminosulfonylaminocarbonyl group optionally substituted with 1 to 2 alkyl groups; and an amino group optionally substituted with 1 to 2 groups independently selected from the group consisting of an alkyl group, an alkanoyl group, and an alkylsulfonyl group.

More specifically, examples of each of the substituents include a hydroxyl group, an oxo group, a cyano group, a methyl group, a carboxymethyl group, a methoxy group, a carboxyl group, a carbamoyl group, a N-methylcarbamoyl group, a N-ethylcarbamoyl group, a N-1-carboxylmethylcarbamoyl group, a N-2-hydroxyethylcarbamoyl group, a N-2-methoxyethylcarbamoyl group, a N-hydroxycarbamoyl group, a N-methyl-N-1-carboxylmethylcarbamoyl group, a N-methyl-N-2-hydroxyethylcarbamoyl group, a N-methyl-N-2-methoxyethylcarbamoyl group, a N,N-dimethylcarbamoyl group, a t-butoxycarbonyl group, an acetyl group, a N,N-dimethylaminosulfonylaminocarbonyl group, a methylsulfonyl group, a methylsulfonylaminocarbonyl group, an aminosulfonyl group, a N,N-dimethylaminosulfonyl group, a N,N-dimethylamino group, a N-methyl-N-acetylamino group, a N-methyl-N-methylsulfonylamino group, a tetrazolyl group, a 4H-[1,2,4]oxadiazol-5-one-2-yl group, a 5-hydroxy-isoxazolyl group, a pyrrolidinecarbonyl group, a 2-carboxylpyrrolidinecarbonyl group, a 2-oxopyrrolidinyl group, and a 1,1'-dioxoisothiazolidin-2-yl group. Especially, a carbamoyl group and a carboxyl group are preferable, and a carboxyl group is particularly preferable.

Examples of $R^5$ include

(1) an alkyl group optionally substituted with a carboxyl group,
(2) an alkenyl group optionally substituted with a carboxyl group,
(3) a 3- to 7-membered monocyclic cycloalkyl group optionally substituted with a carboxyl group,
(4) a 3- to 7-membered monocyclic cycloalkenyl group optionally substituted with a carboxyl group,
(5) a phenyl group optionally substituted with a carboxyl group,
(6) a heteroaryl group optionally substituted with a carboxyl group or an alkyl group optionally substituted with a carboxyl group,
(7) an aliphatic heterocyclic group optionally substituted with 1 to 2 groups independently selected from the group consisting of a hydroxyl group; an oxo group; a cyano group; an alkyl group optionally substituted with a carboxyl group; an alkoxy group; an alkanoyl group; a carboxyl group; an alkoxycarbonyl group; a carbomoyl group optionally substituted with 1 to 2 groups independently selected from the group consisting of an alkyl group and a hydroxyl group (wherein the alkyl is optionally substituted with a hydroxyl group, an alkoxy group, or a carboxyl group); an alkylsulfonylaminocarbonyl group; an aliphatic heterocyclic carbonyl group optionally substituted with a carboxyl group; an amino group optionally substituted with 1 to 2 groups independently selected from the group consisting of an alkyl group, an alkanoyl group, and an alkylsulfonyl group; an aliphatic heterocyclic group optionally substituted with 1 to 2 oxo groups; an alkylsulfonyl group; a tetrazolyl group; and an aminosulfonyl group optionally substituted with 1 to 2 alkyl groups,
(8) an alkoxy group optionally substituted with a group selected from the group consisting of a cyano group; a carboxyl group; a heteroaryl group optionally substituted with a hydroxyl group or an oxo group; an aminosulfonylaminocarbonyl group optionally substituted with 1 to 2 alkyl groups; and an alkylsulfonylaminocarbonyl group,
(9) an amino group optionally substituted with 1 to 2 alkyl groups optionally substituted with a carboxyl group, or
(10) a carbamoyl group.

Specifically, the heteroaryl group of the "(6) heteroaryl group optionally substituted with a carboxyl group or an alkyl group optionally substituted with a carboxyl group" is a 5- to 6-membered monocyclic heteroaryl group including 1 to 4 hetero atoms independently selected from the group consisting of an oxygen atom, a sulfur atom, and a nitrogen atom. The heteroaryl group is, for example, a pyrrolyl group, an imidazolyl group, a pyrazolyl group, an oxazolyl group, an isoxazolyl group, a thiazolyl group, a tetrazolyl group, an oxadiazolyl group, a pyridyl group, a pyrazinyl group, a pyrimidinyl group, a pyridazinyl group, a thiazinyl group, or a triazinyl group, preferably a pyrrolyl group, a pyrazolyl group, an oxazolyl group, a pyridyl group, or a pyrimidinyl group, more preferably, an oxazolyl group or a pyrazolyl group.
The aliphatic heterocyclic ring moiety of the "(7) aliphatic heterocyclic group optionally substituted with 1 to 2 groups independently selected from the group consisting of a hydroxyl group; an oxo group; a cyano group; an alkyl group optionally substituted with a carboxyl group; an alkoxy group; an alkanoyl group; a carboxyl group; an alkoxycarbonyl group; a carbamoyl group optionally substituted with 1 to 2 groups independently selected from the group consisting of an alkyl group and a hydroxyl group (wherein the alkyl is optionally substituted with a hydroxyl group, an alkoxy group, or a carboxyl group); an alkylsulfonylaminocarbonyl group; an aliphatic heterocyclic carbonyl group optionally substituted with a carboxyl group; an amino group optionally substituted with 1 to 2 groups independently selected from the group consisting of an alkyl group, an alkanoyl group, and an alkylsulfonyl group; an aliphatic heterocyclic group optionally substituted with 1 to 2 oxo groups; an alkylsulfonyl group; and an aminosulfonyl group optionally substituted with 1 to 2 alkyl groups" is preferably a 4- to 7-membered monocyclic aliphatic heterocyclic group including 1 to 2 hetero atoms independently selected from the group consisting of an oxygen atom, a sulfur atom, and a nitrogen atom, and specific examples thereof include an azetidinyl group, a pyrrolidinyl group, a tetrahydrofuranyl group, an imidazolinyl group, a thiazolidinyl group, an isothiazolidinyl group, a piperidinyl group, a piperazinyl group, a morpholinyl group, a thiomorpholinyl group, a tetrahydropyranyl group, a homopiperazinyl group, a homomorpholinyl group, a 3-azabicyclo[3.1.0]hexyl group, and an octahydropyrrolo[3,4-c]pyrrolyl group. More preferable are an azetidinyl group, a pyrrolidinyl group, a piperidinyl group, a piperazinyl group, a morpholinyl group, a thiomorpholinyl group, and a 3-azabicyclo[3.1.0]hexyl group.
The aliphatic heterocyclic ring of the "aliphatic heterocyclic carbonyl group optionally substituted with a carboxyl group" is preferably a 4- to 7-membered monocyclic aliphatic heterocyclic ring including at least one nitrogen atom and optionally further including one hetero atom selected from the group consisting of an oxygen atom, a sulfur atom, and a nitrogen atom, and examples thereof include an azetidinyl group, a pyrrolidinyl group, an imidazolinyl group, a thiazolidinyl group, an isothiazolidinyl group, a piperidinyl group, a piperazinyl group, a morpholinyl group, a thiomorpholinyl group, a homopiperazinyl group, or a homomorpholinyl group. A pyrrolidinyl group is particularly preferable. The aliphatic heterocyclic group of the "aliphatic heterocyclic group optionally substituted with 1 to 2 oxo groups" is preferably a 4- to 7-membered monocyclic aliphatic heterocyclic group including 1 to 2 hetero atoms independently selected from the group consisting of an oxygen atom, a sulfur atom, and a nitrogen atom, and specific examples thereof include an azetidinyl group, a pyrrolidinyl group, a tetrahydrofuranyl group, an imidazolinyl group, a thiazolidinyl group, an isothiazolidinyl group, a piperidinyl group, a piperazinyl group, a morpholinyl group, a thiomorpholinyl group, a tetrahydropyranyl group, a homopiperazinyl group, or a homomorpholinyl group. A pyrrolidinyl group or an isothiazolidinyl group is particularly preferable.

The heteroaryl group of the "(8) alkoxy group optionally substituted with a group selected from the group consisting of a cyano group; a carboxyl group; a heteroaryl group optionally substituted with a hydroxyl group or a oxo group; an aminosulfonylaminocarbonyl group optionally substituted with 1 to 2 alkyl groups; and an alkylsulfonylaminocarbonyl group" is preferably a 5- to 6-membered monocyclic heteroaryl group including 1 to 4 hetero atoms independently selected from the group consisting of an oxygen atom, a sulfur atom, and a nitrogen atom, and specific example thereof include a pyrrolyl group, an imidazolyl group, a pyrazolyl group, an oxazolyl group, an isoxazolyl group, a thiazolyl group, a tetrazolyl group, an oxadiazolyl group, a pyridinyl group, a pyrazinyl group, a pyrimidinyl group, a pyridazinyl group, a thiazinyl group, and a triazinyl group. A tetrazolyl group, an oxadiazolyl group, and an isoxazolyl group are particularly preferable.

Particularly suitable examples of $R^5$ include an alkyl group substituted with a carboxyl group, an alkenyl group substituted with a carboxyl group, a cycloalkyl group substituted with a carboxyl group, a cycloalkenyl group substituted with a carboxyl group, an aryl group substituted with a carboxyl group, a heteroaryl group substituted with a carboxyl group, an aliphatic heterocyclic group substituted with a carboxyl group, an alkoxy group substituted with a carboxyl group, an amino group optionally substituted with 1 to 2 alkyl groups substituted with a carboxyl group, or a carbamoyl group. Especially, an aliphatic heterocyclic group substituted with a carboxyl group is preferable, and a piperidinyl group substituted with a carboxyl group is particularly preferable.

In "a hydrogen atom, a halogen atom, a cyano group, an alkyl group, a haloalkyl group, and a haloalkoxy group" represented by $R^6$ or $R^7$, the halogen atom is preferably a fluorine atom or a chlorine atom. Suitable examples of the "alkyl group" include a methyl group, an ethyl group, and an i-propyl group. The "haloalkyl group" is preferably, for example, a trifluoromethyl group or a difluoromethyl group. The "haloalkoxy group" is preferably, for example, a trifluoromethoxy group.

$R^6$ or $R^7$ is preferably, for example, a hydrogen atom, a fluorine atom, a chlorine atom, a methyl group, an ethyl group, an i-propyl group, a trifluoromethyl group, difluoromethyl group, or a trifluoromethoxy group.

In particular, a case is preferable where $R^6$ is a fluorine atom, a chlorine atom, a methyl group, an i-propyl group, a trifluoromethyl group, a difluoromethyl group, or a trifluoromethoxy group, and $R^7$ is a hydrogen atom. A case is especially preferable where $R^6$ is a fluorine atom, a chlorine atom, a methyl group, or a trifluoromethyl group, and $R^7$ is a hydrogen atom. A case is also preferable where both $R^6$ and $R^7$ are each a fluorine atom.

A preferable embodiment of the compound of the general formula [I] includes a compound or a pharmaceutically acceptable salt or cocrystal thereof, wherein, in the general formula [I]:

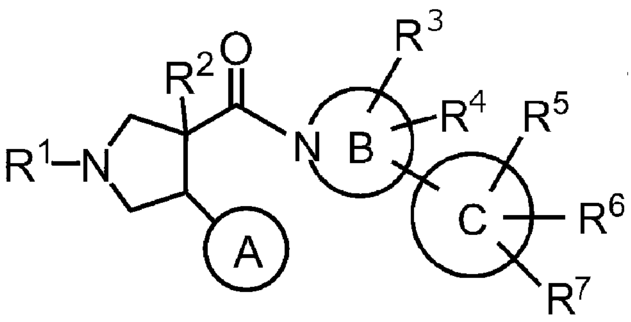

[I]

ring A represents an optionally substituted aryl group or an optionally substituted heteroaryl group,

wherein each substituent in the optionally substituted aryl group and the optionally substituted heteroaryl group corresponds to 1 to 3 groups independently selected from the group consisting of a halogen atom, an alkyl group, a haloalkyl group, a cycloalkyl group, an alkoxy group, a haloalkoxy group, and an alkyleneoxy group;
$R^1$ represents an optionally substituted alkyl group, an optionally substituted cycloalkyl group, an optionally substituted aliphatic heterocyclic group, an optionally substituted and optionally partially hydrogenated aryl group, an optionally substituted heteroaryl group, or a carbamoyl group optionally substituted with 1 to 2 alkyl groups,
wherein a substituent in the optionally substituted alkyl group corresponds to 1 to 3 groups independently selected from the group consisting of a halogen atom; a hydroxyl group; an oxo group; a cyano group; a cycloalkyl group; an alkoxy group; an alkanoyl group; a carbamoyl group optionally substituted with 1 to 2 alkyl groups; an aliphatic heterocyclic group; an aliphatic heterocyclic carbonyl group optionally substituted with 1 to 2 groups independently

selected from the group consisting of a halogen atom, an alkyl group, a haloalkyl group, and an alkoxyalkyl group; an alkylsulfonyl group; an aliphatic heterocyclic sulfonyl group; and an alkyleneoxy group, and
each substituent in the optionally substituted cycloalkyl group, the optionally substituted aliphatic heterocyclic group, the optionally substituted and optionally partially hydrogenated aryl group, and the optionally substituted heteroaryl group corresponds to 1 to 3 groups independently selected from the group consisting of a halogen atom; a hydroxyl group; an oxo group; a cyano group; an alkyl group; a haloalkyl group; a cycloalkyl group; an alkoxy group; a hydroxyalkyl group; an alkoxyalkyl group; an alkanoyl group; a carbamoyl group optionally substituted with 1 to 2 alkyl groups; an aliphatic heterocyclic group; an aliphatic heterocyclic carbonyl group optionally substituted with 1 to 2 groups independently selected from the group consisting of a halogen atom, an alkyl group, a haloalkyl group, and an alkoxyalkyl group; an alkylsulfonyl group; an aliphatic heterocyclic sulfonyl group; and an alkyleneoxy group;

$R^2$ represents a halogen atom, an alkyl group, or an alkoxy group;
ring B represents a nitrogen-containing aliphatic heterocyclic group optionally partially including a double bond,
ring C represents an aryl group or a heteroaryl group,
$R^3$ and $R^4$ each independently represent a group selected from the group consisting of a hydrogen atom, a halogen atom, a cyano group, an alkyl group, a haloalkyl group, a cyanoalkyl group, a hydroxyalkyl group, an alkoxyalkyl group, a carboxyl group, a carbamoyl group optionally substituted with 1 to 2 alkyl groups, and an alkoxy group;
$R^5$ represents an optionally substituted alkyl group, an optionally substituted alkenyl group, an optionally substituted cycloalkyl group, an optionally substituted cycloalkenyl group, an optionally substituted aryl group, an optionally substituted heteroaryl group, an optionally substituted aliphatic heterocyclic group, an optionally substituted alkoxy group, an amino group optionally substituted with 1 to 2 alkyl groups optionally substituted with a carboxyl group, or a carbamoyl group optionally substituted with 1 to 2 alkyl groups optionally substituted with a carboxyl group,

wherein a substituent in the optionally substituted alkyl group corresponds to 1 to 2 groups independently selected from the group consisting of a hydroxyl group; an oxo group; a cyano group; an alkoxy group; an alkanoyl group; a carboxyl group; an alkoxycarbonyl group; an aliphatic heterocyclic carbonyl group optionally substituted with a carboxyl group; a heteroaryl group optionally substituted with a hydroxyl group or a oxo group; an aliphatic heterocyclic group optionally substituted with 1 to 2 oxo groups; a carbamoyl group optionally substituted with 1 to 2 groups independently selected from the group consisting of an alkyl group (the alkyl moiety is optionally substituted with a hydroxyl group, an alkoxy group, or a carboxyl group) and a hydroxyl group; an alkylsulfonyl group; an aminosulfonyl group optionally substituted with 1 to 2 alkyl groups; an aminosulfonylaminocarbonyl group optionally substituted with 1 to 2 alkyl groups; an alkylsulfonylaminocarbonyl group; and an amino group optionally substituted with 1 to 2 groups independently selected from the group consisting of an alkyl group, an alkanoyl group, and an alkylsulfonyl group, and
each substituent in the optionally substituted alkenyl group, the optionally substituted cycloalkyl group, the optionally substituted cycloalkenyl group, the optionally substituted aryl group, the optionally substituted heteroaryl group, the optionally substituted aliphatic heterocyclic group, and the optionally substituted alkoxy group corresponds to 1 to 2 groups independently selected from the group consisting of a hydroxyl group; an oxo group; a cyano group; an alkyl group optionally substituted with a carboxyl group; an alkoxy group; an alkanoyl group; a carboxyl group; an alkoxycarbonyl group; an aliphatic heterocyclic carbonyl group optionally substituted with a carboxyl group; a heteroaryl group optionally substituted with a hydroxyl group or a oxo group; an aliphatic heterocyclic group optionally substituted with 1 to 2 oxo groups; a carbamoyl group optionally substituted with 1 to 2 groups independently selected from the group consisting of an alkyl group (the alkyl moiety is optionally substituted with a hydroxyl group, an alkoxy group, or a carboxyl group) and a hydroxyl group; an alkylsulfonyl group; an aminosulfonyl group optionally substituted with 1 to 2 alkyl groups; an aminosulfonylaminocarbonyl group optionally substituted with 1 to 2 alkyl groups; an alkylsulfonylaminocarbonyl group; and an amino group optionally substituted with 1 to 2 groups independently selected from the group consisting of an alkyl group, an alkanoyl group, and an alkylsulfonyl group; and
$R^6$ and $R^7$ each independently represent a group selected from the group consisting of a hydrogen atom, a halogen atom, a cyano group, an alkyl group, a haloalkyl group, and a haloalkoxy group.

Another preferable embodiment of the compound of the general formula [I] includes a compound or a pharmaceutically acceptable salt or cocrystal thereof, wherein, in the general formula [I], ring A is a phenyl group optionally substituted with an alkoxy group,

$R^1$ is a 5- to 6-membered monocyclic cycloalkyl group optionally substituted with a group selected from the group consisting of an alkoxy group and a cyano group,

$R^2$ is a halogen atom or an alkoxy group,
ring B is a pyrrolidinyl group, and $R^3$ is an alkoxyalkyl group and $R^4$ is a hydrogen atom or a halogen atom,
ring C is a phenyl group,
$R^5$ is a piperidinyl group substituted with a carboxyl group,
$R^6$ is a halogen atom or a haloalkyl group, and
$R^7$ is a hydrogen atom.

A more specific preferable embodiment of the compound of the general formula [I] includes a compound, or a pharmaceutically acceptable salt or cocrystal thereof, selected from the group consisting of:

1-{2-[(3S,5S)-1-{[(3R,4R)-1-(trans-4-ethoxycyclohexyl)-3-methoxy-4-(4-methoxyphenyl)pyrrolidin-3-yl]carbonyl}-5-(ethoxymethyl)pyrrolidin-3-yl]-5-(trifluoromethyl)phenyl}piperidine-4-carboxylic acid;
1-{2-[(3S,SS)-1-{[(3R,4R)-3-fluoro-4-(4-methoxyphenyl)-1-(2-methylpyridin-4-yl)pyrrolidin-3-yl]carbonyl}-5-(methoxymethyl)pyrrolidin-3-yl]-5-(trifluoromethyl)phenyl}piperidine-4-carboxylic acid;
1-{2-[(3S,SS)-1-{(3R,4R)-3-fluoro-4-(4-methoxyphenyl)-1-(2-methylpyrimidin-4-yl)pyrrolidin-3-yl]carbonyl}-5-(methoxymethyl)pyrrolidin-3-yl]-5-(trifluoromethyl)phenyl}piperidine-4-carboxylic acid;
1-{5-fluoro-2-[(3S,5S)-1-{[(3R,4R)-3-fluoro-1-(trans-4-methoxycyclohexyl)-4-(4-methoxyphenyl)pyrrolidin-3-yl]carbonyl}-5-(methoxymethyl)pyrrolidin-3-yl]phenyl}piperidine-4-carboxylic acid;
1-{2-[(3S,5S)-1-{[(3R,4R)-1-(trans-4-ethoxycyclohexyl)-3-methoxy-4-(4-methoxyphenyl)pyrrolidin-3-yl]carbonyl}-5-(methoxymethyl)pyrrolidin-3-yl]-5-(trifluoromethyl)phenyl}piperidine-4-carboxylic acid;
1-{2-[(3S,5S)-1-{[(3R,4R)-1-(trans-4-cyanocyclohexyl)-3-fluoro-4-(4-methoxyphenyl)pyrrolidin-3-yl]carbonyl}-5-(methoxymethyl)pyrrolidin-3-yl]- 5-(trifluoromethyl)phenyl}piperidine-4-carboxylic acid;
1-{2-[(3S,4S)-1-{[(3R,4R)-1-(trans-4-ethoxycyclohexyl)-3-methoxy-4-(4-methoxyphenyl)pyrrolidin-3-yl]carbonyl}-4-fluoro-4-(methoxymethyl)pyrrolidin-3-yl]-5-(trifluoromethyl)phenyl}piperidine-4-carboxylic acid;
1-{2-[(3S,4R)-1-{[(3R,4R)-1-(trans-4-ethoxycyclohexyl)-3-methoxy-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid;
1-{2-[(3S,5S)-1-{[(3R,4R)-1-cyclohexyl-3-fluoro-4-(4-methoxyphenyl)pyrrolidin-3- yl]carbonyl}-5-(methoxymethyl)pyrrolidin-3-yl]-5-(trifluoromethyl)phenyl}piperidine-4-carboxylic acid;
1-{2-[(3S,SS)-1-{[(3R,4R)-3-fluoro-4-(4-methoxyphenyl)-1-(tetrahydro-2H-pyran-4-yl)pyrrolidin-3-yl]carbonyl}-5-(methoxymethyl)pyrrolidin-3-yl]-5-(trifluoromethyl)phenyl}piperidine-4-carboxylic acid;
1-{2-[(3S,5S)-1-{[(3R,4R)-3-fluoro-1-(trans-4-methoxycyclohexyl)-4-(4-methoxyphenyl)pyrrolidin-3-yl]carbonyl}-5-(methoxymethyl)pyrrolidin-3-yl]-5-(trifluoromethyl)phenyl}piperidine-4-carboxylic acid;
1-{2-[(3S,5S)-1-{[(3R,4R)-1-cyclopentyl-3-fluoro-4-(4-methoxyphenyl)pyrrolidin-3- yl]carbonyl}-5-(methoxymethyl)pyrrolidin-3-yl]-5-(trifluoromethyl)phenyl}piperidine-4-carboxylic acid;
1-{2-[(3S,4R)-1-{[(3R,4R)-3-fluoro-4-(4-methoxyphenyl)-1-(2-methylpyridin-4-yl)pyrrolidin-3-yl]carbonyl}-4-(methoxymethyl)pyrrolidin-3-yl]-5-(trifluoromethyl)phenyl}piperidine-4-carboxylic acid;
1-{2-[(3S,4S)-4-fluoro-1-{[(3R,4R)-3-fluoro-1-(trans-4-methoxycyclohexyl)-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid;
1-{5-chloro-2-[(3S,4R)-1-{[(3R,4R)-3-fluoro-1-(trans-4-methoxycyclohexyl)-4-(4-methoxyphenyl)pyrrolidin-3-yl]carbonyl}-4-(methoxymethyl)pyrrolidin-3-yl]phenyl}piperidine-4-carboxylic acid;
1-{2-[(3S,4R)-4-(cyanomethyl)-1-{[(3R,4R)-1-cyclopentyl-3-fluoro-4-(4-methoxyphenyl)pyrrolidin-3-yl]carbonyl}pyrrolidin-3-yl]-5-(trifluoromethyl)phenyl}piperidine-4-carboxylic acid;
1-{2-[(3S,4R)-1-{[(3R,4R)-3-fluoro-1-(trans-4-methoxycyclohexyl)-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid;
1-[2-(1-{[(3R,4R)-3-fluoro-1-(trans-4-methoxycyclohexyl)-4-(4-methoxyphenyl)pyrrolidin-3-yl]carbonyl}piperidin-4-yl)-5-(trifluoromethyl)phenyl]piperidine-4-carboxylic acid;
1-{2-[(3S,4R)-1-{[(3R,4R)-1-cyclopentyl-3-fluoro-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid; and
1-{2-[(3S)-1-{[(3R,4R)-3-fluoro-1-(trans-4-methoxycyclohexyl)-4-(4-methoxyphenyl)pyrrolidin-3-yl]carbonyl}pyrrolidin-3-yl]-5-(trifluoromethyl)phenyl}piperidine-4-carboxylic acid.

While the compound of the general formula [I], when has an asymmetric carbon atom, can be present as a plurality of stereoisomers (namely, diastereoisomer and optical isomer) based on the asymmetric carbon atom, the compound in the present invention encompasses both any one of such stereoisomers and a mixture thereof. The compound of the general formula [I] may also be represented with a bond indicated by the following wavy line in order to suppose that the compound is a mixture of a plurality of such stereoisomers.

The compound of the general formula [I] can encompass cis- and trans-isomers as geometric isomers, or, when has axial asymmetry in its molecule, can encompass any isomer based on the axial asymmetry, and the compound encompasses both any one of such isomers and a mixture thereof.

The compound of the general formula [I] encompasses a compound labelled with an isotope (for example, $^{3}H$, $^{13}C$, $^{14}C$, $^{15}N$, $^{18}F$, $^{32}P$, $^{35}S$, or $^{125}I$) and a compound replaced with deuterium.

The compound of the general formula [I], or the pharmaceutically acceptable salt or cocrystal thereof is described in Patent Document 7, and can be produced by a method described in Patent Document 7.

<Medical use>

The compound of the general formula [I], or the pharmaceutically acceptable salt or cocrystal thereof has an excellent MC1R agonist activity, and thus promotes melanin production in melanin production cells and promotes skin pigmentation, also in vivo. Accordingly, a medicament comprising the compound of the general formula [I], or the pharmaceutically acceptable salt or cocrystal thereof, as an active ingredient, is useful for treating or preventing a condition expected to be ameliorated mainly by promotion of melanin production through the activation of MC1R, for example, photodermatoses (also called photosensitivity), hypopigmentary disease, and adverse effects of phototherapy.

The photodermatoses can be prevented and/or the symptom thereof can be ameliorated and/or inhibited from progressing, by promotion of melanin production in melanin production cells in the skin and an increase in skin pigmentation, and thus protection of the skin from light rays such as ultraviolet light and solar light.

Examples of the photodermatoses include photoallergic dermatitis, hydroa vacciniforme, DNA repair disorder, and porphyria (excluding protoporphyria).

Examples of the photoallergic dermatitis include solar urticaria, polymorphous light eruption, chronic actinic dermatitis, photoallergic contact dermatitis, and photoallergic drug eruption.

Examples of the DNA repair disorder include xeroderma pigmentosum, Bloom syndrome, Werner syndrome, and Rothmund-Thomson syndrome, Trichothiodystrophy, and UV-sensitive syndrome.

Examples of the porphyria (excluding protoporphyria) include congenital erythropoietic porphyria, variegate porphyria, acute intermittent porphyria, porphyria cutanea tarda, and hereditary coproporphyria.

The hypopigmentary disease can be prevented and/or the symptom thereof can be ameliorated and/or inhibited from progressing, by promotion of melanin production in melanin production cells in the skin and an increase in skin pigmentation, and thus protection of the skin from light rays such as ultraviolet light and solar light.

Examples of the hypopigmentary disease include congenital hypopigmentary diseases such as oculocutaneous albinism, piebaldism, nevus depigmentosus, hypomelanosis of Ito, phenylketonuria, tuberous sclerosis, dyschromatosis symmetrica hereditaria, Waardenburg syndrome, Hermansky-Pudlak syndrome, Chediak-Higashi syndrome, Griscelli syndrome, and Tietz syndrome, and acquired hypopigmentary diseases such as leukoderma Sutton, Vogt-Koyanagi-Harada syndrome, leukoderma senile, leukoderma after sea bathing, and leukoderma syphiliticum.

While phototherapy is used for treating, for example, inflammatory skin disease, leukoderma, and alopecia areata, and, for example, narrow band UVB therapy, excimer light therapy, and PUVA therapy are known, examples of short-term adverse effects include photosensitive symptoms and phototoxic symptoms, such as rednes, sunburn, and heat of any portion radiated, and examples of long-term adverse effects include cancers such as skin cancer, and photoaging such as pigmented spots and wrinkles. The compound of the general formula [I], or the pharmaceutically acceptable salt or cocrystal thereof can protect the skin from such adverse effects of phototherapy to exert the effect of prevention and the effect of treatment.

The following Literature "10.2 Skin Color and Skin Phototypes" describes an increase in melanin, which not only can allow the skin to be protected from light rays, but also can suppress adverse effects such as cancerization of the skin due to light rays.

"Photodermatoses in Pigmented Skin", Vinod Kumar Sharma et al., Photochem Photobiol Sci, 12 (1), 65-77 Jan 2013

The compound of the general formula [I] or the pharmaceutically acceptable salt or cocrystal thereof can promote melanin production in melanocytes present in hair bulbs, to thereby allow for incorporation of melanin produced, into adjacent hair matrix cells, and thus blackening of hair, and is effective for preventing and/or ameliorating gray hair.

There are several methods for evaluating the efficacy of treatment for xeroderma pigmentosum, a photosensitive DNA repair disorder, including: in vitro studies such as the unscheduled DNA synthesis test after UV irradiation and the UV lethality susceptibility test (colony formation method), as reported by Moriwaki et al. in their practice guidelines (J (J Dermatol 2017, 44, 1087); an in vivo study in which Xpa knockout mice were irradiated with UV light to induce skin inflammation and skin cancer as reported by Kunisada et al. (J Invest Dermatol 2017, 137, 1975), and the Polr2aKR/KR/Xpa-/- transgenic mouse model as reported by Nakazawa et al. (Cell 2020, 180, 1228).

The compound of the general formula [I] can be subjected to medical use even in the form of a free compound or in the form of a pharmaceutically acceptable salt or cocrystal thereof.

The compound of the general formula [I], or the pharmaceutically acceptable salt or cocrystal thereof encompasses not only an inner salt and an adduct thereof, but also, for example, all a solvate, a hydrate, and a crystal polymorph thereof.

Examples of the pharmaceutically acceptable salt, cocrystal, inner salt, and adduct include one including any inorganic acid such as hydrochloric acid, sulfuric acid, phosphoric acid, or hydrobromic acid, and one including any organic acid such as acetic acid, fumaric acid, oxalic acid, citric acid, methanesulfonic acid, benzenesulfonic acid, p-toluenesulfonic acid, or maleic acid.

The cocrystal of the compound of the general formula [I], here used, can be preferably a cocrystal of the compound of the general formula [I] and phosphoric acid. In particular, a cocrystal of a compound A and phosphoric acid can be obtained according to a method described in Patent Document 8.

The compound of the general formula [I], or the pharmaceutically acceptable salt or cocrystal thereof may be administered to a patient as it is singly or in combination of two or more kinds thereof, and preferably, the compound of the general formula [I], or the pharmaceutically acceptable salt or cocrystal thereof can be mixed with any medically and pharmaceutically acceptable additive and the resultant can be provided as a formulation whose form is well known by those skilled in the art.

Such any medically and pharmaceutically acceptable additive here used can be any of appropriate excipient, disintegrant, binder, lubricant, coating agent, pigment, diluent, base material, tonicity agent, and the like which are usually used in production of a medicament. For example, the excipient here used can be, for example, glucose, lactose, D-mannitol, starch, or crystalline cellulose, the disintegrant here used can be, for example, carboxymethylcellulose, starch, or carboxymethylcellulose calcium, the binder here used can be, for example, hydroxypropylcellulose, hydroxypropylmethylcellulose, polyvinyl pyrrolidone, or gelatin, the lubricant here used can be, for example, magnesium stearate or talc, the coating agent here used can be, for example, hydroxypropylmethylcellulose, white soft sugar, polyethylene glycol, or titanium oxide, and the base material here used can be, for example, vaseline, liquid paraffin, polyethylene glycol, gelatin, kaolin, glycerin, purified water, or hard fat. Any additive for formulations, for example, a dissolving agent or a dissolution aid which can constitute an aqueous or use-time dissolution type injection, such as distilled water for injection, saline, or propylene glycol; a tonicity agent such as glucose, sodium chloride, D-mannitol, or glycerin; or a pH-regulator such as an inorganic acid, an organic acid, an inorganic base, or an organic base can be used in any formulation suitable for injections or infusion.

The compound of the general formula [I], or the pharmaceutically acceptable salt or cocrystal thereof, can be combined with the above additive to thereby prepare an appropriate administration form (for example, powder, injection product, tablet, capsule, or topical external preparation), and thereafter the resultant can be administered to a patient (human or animal) according to an appropriate administration method (for example, intravenous administration, oral administration, transdermal administration, or topical administration) depending on the administration form. In particular, oral administration is preferable.

The amount of dosage is determined depending on age, body weight, general health conditions, sex, diet, the administration time, the administration method, the excretion rate, a combination of drugs, and the severity of the disease condition of a patient being treated in administration, or in consideration of these or other factors.

The amount of dosage of the medicament including the compound of the general formula [I], or the pharmaceutically acceptable salt or cocrystal thereof is not particularly limited as long as it can allow for not only low toxicity and safe use, but also exertion of the effect of treatment or the effect of prevention of diseases and conditions, such as photodermatoses, hypopigmentary disease, adverse effects of phototherapy, and gray hair, and the amount is, for example, 1 to 1000 mg/day, preferably 10 to 500 mg/day as the amount of the compound of the general formula [I]. The amount of the compound of the general formula [I], or the pharmaceutically acceptable salt or cocrystal thereof is, for example, preferably 50 to 500 mg/day, more preferably 80 to 400 mg/day, particularly preferably 100 to 300 mg/day, for example, 100 mg/day, 150 mg/day, 200 mg/day, 250 mg/day, 300 mg/day, or any amount of dosage therebetween, as the amount of the compound of the general formula [I], in order that skin pigmentation is more certainly promoted and the effect of treatment or the effect of prevention of diseases and conditions, such as photodermatoses, hypopigmentary disease, adverse effects of phototherapy, and gray hair is exerted.

In particular, oral administration is preferable, and the medicament comprising the compound of the general formula [I], or the pharmaceutically acceptable salt or cocrystal thereof is orally administered in an amount of dosage of, for example, 50 to 500 mg/day, preferably 80 to 400 mg/day, more preferably 100 to 300 mg/day, specifically, 100 mg/day, 150 mg/day, 200 mg/day, 250 mg/day, 300 mg/day, or any amount therebetween, as the amount of the compound of the general formula [I].

EXAMPLES

Hereinafter, the present invention will be specifically described with reference to Examples, but aspects of the present invention are not limited to aspects of the following Examples.
The compound A used in Examples was the following compound.
Cocrystal of 1-{2-[(3 S,4R)-1-{[(3R,4R)-1-cyclopentyl-3-fluoro-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid with phosphoric acid
The compound B used in Examples was the following compound.

1-{2-[(3S,5S)-1-{[(3R,4R)-1-(trans-4-ethoxycyclohexyl)-3-methoxy-4-(4-methoxyphenyl)pyrrolidin-3-yl]carbonyl}-5-(methoxymethyl)pyrrolidin-3-yl]-5-(trifluoromethyl)phenyl}piperidine-4-carboxylic acid, 2 hydrochloride
1-{2-[(3S,4R)-1-{[(3R,4R)-1-Cyclopentyl-3-fluoro-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid was produced according to a method described in Patent Document 7, and the cocrystal thereof with phosphoric acid was produced according to the following method.

In other words, a solution of potassium carbonate (3.4 kg) in water (77.0 L) and water (19.3 L) were sequentially added at 20 to 30°C to a suspension of 1-{2-[(3S,4R)-1-{[(3R,4R)-1-cyclopentyl-3-fluoro-4-(4-methoxyphenyl)pyrrolidin-3- yl]carbonyl}-4-(methoxymethyl)pyrrolidin-3-yl]-5-(trifluoromethyl)phenyl}piperidine-4-carboxylic acid 1/2 ethane-1,2-disulfonic acid (19.3 kg) in ethyl acetate (86.6 kg), and the resulting mixture was stirred for 10 minutes. After the mixture was left to still stand, an aqueous layer was removed, and an organic layer was washed with water (96.3 L) twice. After the organic layer was concentrated to 35 L, ethanol (75.9 kg) was added thereto and the resultant was concentrated to 35 L. After dilution with ethanol (30.3 kg), an insoluble fraction was collected by filtration, and washed with ethanol (75.6 kg). The filtrate was concentrated to 35 L, and diluted with ethanol (17.9 kg). An aqueous 24% sodium hydroxide solution (6.1 kg) and water (15.6 kg) were sequentially added thereto at 20 to 30°C, and the resulting mixture was stirred at 20 to 30°C for 5 hours. A solution of phosphoric acid (8.5 kg) in water (28.9 L) and water (115.5 L) were sequentially added thereto at 20 to 40°C. The compound A (0.48 kg) was added thereto as a seed crystal at 30 to 40°C, and the resultant was stirred for 19.5 hours and then cooled to 20°C. A solid was collected by filtration, and the solid was washed with water (96.3 L). The solid was dried at 50°C or less, and thereafter pulverized, thereby obtaining the compound A (17.5 kg).
The compound A obtained was identified with IR.
1-{2-[(3 S,5S)-1-{[(3R,4R)-1-(trans-4-ethoxycyclohexyl)-3-methoxy-4-(4-methoxyphenyl)pyrrolidin-3-yl]carbonyl}-5-(methoxymethyl)pyrrolidin-3-yl]-5-(trifluoromethyl)phenyl}piperidine-4-carboxylic acid, 2 hydrochloride (Compound B) was produced according to a method described in Patent Document 7, and the 2 hydrochloride thereof was produced according to the conventional method.

## Example 1

[Measurement of agonist activities on human and mouse MC1R (measurement of cAMP)]

Human embryonic kidney cells 293 (HEK293 cells) stably expressing human MC1R or mouse MC1R were used. A solution containing each concentration of the compound was admixed with cAMP assay buffer (HBSS (Hank's Balanced Salt Solution) containing 10 mM HEPES and 1% BSA), and dispensed into a 96 well black half area plate. Cells expressing human MC1R ($3 \times 10^3$ cells/well) or cells expressing mouse MC1R ($18 \times 10^3$ cells/well) were suspended in cAMP assay buffer containing 0.5 mM IBMX, the resulting suspension was dispensed into the 96 well plate, thereafter admixed, and left to still stand at 37°C for 30 minutes, and thereafter the intracellular cAMP concentration was measured by a fluorescence method using Envision (ex. 320 nm, em. 665 nm and 615 nm). The resulting data was used to calculate the quantitative value of the cAMP concentration, with Prism 5.02, from the ratio value (Measurement value at 665 nm/Measurement value at 615 nm $\times$ 10000), and each maximum amount Emax of response and each EC50 (concentration at which a response corresponding to one-half of Emax with respect to αMSH was exhibited) with respect to αMSH, the compound A and NDP-αMSH ([Nle4,D-Phe7]-α-MSH) were calculated according to non-linear regression.
The results are shown in Table 1. The compound A exhibited an agonist activity against human MC1R and mouse MC1R.

[Table 1]

| | $EC_{50}$ (nmol/L) | |
|---|---|---|
| | HMC1R | mMC1R |
| $\alpha$ MSH | 0.203 (0. 166-0.250) | 3.17 (2.01-5.01) |
| NDP- $\alpha$ MSH | 0.0564 (0. 0426-0. 0748) | 0.0524 (0. 0274-0. 100) |
| Compound A | 8. 16 (6. 74-9. 88) | 1. 14 (0. 259-5. 06) |

Example 2

[Evaluation of binding selectivity to human MCR subtypes]

The respective binding abilities of the compound A to MC1R, MC3R, MC4R, and MC5R were evaluated by a radioactively labeled ligand ([$^{125}$I]NDP-$\alpha$-MSH) binding assay.

<Measurement method>

Evaluation of respective actions on MC1R, MC3R, MC4R, and MC5R was performed in experimental conditions described below.

MC1R:

I. Buffer:

1. Incubation buffer: 25 mmol/L HEPES, pH 7.0, 100 mmol/L NaCl, 1 mmol/L 1,10-Phenanthroline, 1.5 mmol/L $CaCl_2$, 1 mmol/L $MgSO_4$, one Complete™ protease inhibitor tablet/100 mL
2. Wash buffer: 50 mmol/L Tris-HCl, pH 7.4

II. Preparation of receptor:

Human recombinant MC1R (PerkinElmer) was dissolved in incubation buffer.

III. Method:

Reaction liquid

Test substance solution or vehicle (1% DMSO*): 2.2 $\mu$L
0.04 nmol/L* [$^{125}$I]NDP-$\alpha$-MSH in 20% BSA: 20 $\mu$L
8.33 $\mu$g/mL receptor solution: 200 $\mu$L
* Final concentration in evaluation

1. The test substance and the receptor solution were mixed, and thereafter the resulting mixture was incubated together with [$^{125}$I]NDP-$\alpha$-MSH at 37°C for 120 minutes.
2. A composite of receptor/[$^{125}$I]NDP-$\alpha$-MSH was recovered by suction and filtration with a GFB fitter mat. Thereafter, the composite was washed with wash buffer.
3. The amount of radioactivity was measured with a scintillation counter (Top Count NXT, PerkinElmer).

MC3R:

I. Buffer:

1. Incubation buffer: 25 mmol/L HEPES, pH 7.0, 100 mmol/L NaCl, 1 mmol/L 1,10-Phenanthroline, 1.5 mmol/L $CaCl_2$, 1 mmol/L $MgSO_4$, one Complete™ protease inhibitor tablet/100 mL
2. Wash buffer: 50 mmol/L Tris-HCl, pH 7.4

II. Preparation of receptor:

Human recombinant MC3R (PerkinElmer) was dissolved in incubation buffer.

III. Method:

Reaction liquid

Test substance solution or vehicle (1% DMSO*): 2.2 μL
0.035 nmol/L* [$^{125}$I]NDP-α-MSH in 20% BSA: 20 μL
24 μg/mL receptor solution: 200 μL
* Final concentration in evaluation

1. The test substance and the receptor solution were mixed, and thereafter the resulting mixture was incubated together with [$^{125}$I]NDP-α-MSH at 37°C for 60 minutes.
2. A composite of receptor/[$^{125}$I]NDP-α-MSH was recovered by suction and filtration with a GFB fitter mat. Thereafter, the composite was washed with wash buffer.
3. The amount of radioactivity was measured with a scintillation counter (Top Count NXT, PerkinElmer).

MC4R:

I. Buffer:

1. Incubation buffer: 25 mmol/L HEPES, pH 7.0, 1.5 mmol/L $CaCl_2$, 1 mmol/L $MgSO_4$, 100 mmol/L NaCl, 1 mmol/L 1,10-Phenanthroline, one Complete™ protease inhibitor tablet/100 mL
2. Wash buffer: 50 mmol/L Tris-HCl, pH 7.4

II. Preparation of receptor:

Human recombinant MC4R (PerkinElmer) was dissolved in incubation buffer.

III. Method:

Reaction liquid

Test substance solution or vehicle (1% DMSO*): 2.2 μL
0.02 nmol/L* [$^{125}$I]NDP-α-MSH in 20% BSA: 20 μL
6.67 μg/mL receptor solution: 200 μL
* Final concentration in evaluation

1. The test substance and the receptor solution were mixed, and thereafter the resulting mixture was incubated together with [$^{125}$I]NDP-α-MSH at 37°C for 120 minutes.
2. A composite of receptor/[$^{125}$I]NDP-α-MSH was recovered by suction and filtration with a GFB fitter mat. Thereafter, the composite was washed with wash buffer.
3. The amount of radioactivity was measured with a scintillation counter (Top Count NXT, PerkinElmer).

MC5R:

I. Buffer:

1. Incubation buffer: 25 mmol/L HEPES, pH 7.0, 100 mmol/L NaCl, 1 mmol/L 1,10-Phenanthroline, 1.5 mmol/L $CaCl_2$, 1 mmol/L $MgSO_4$, one complete™ protease inhibitor tablet/100 mL
2. Wash buffer: 50 mmol/L Tris-HCl, pH 7.4

II. Preparation of receptor:

Human recombinant MC5R (PerkinElmer) was dissolved in incubation buffer.

III. Method:

Reaction liquid

Test substance solution or vehicle (1% DMSO*): 2.2 μL
0.035 nmol/L* [$^{125}$I]NDP-α-MSH in 20% BSA: 20 μL
24 μg/mL receptor solution: 200 μL
* Final concentration in evaluation

1. The test substance and the receptor solution were mixed, and thereafter the resulting mixture was incubated together with [$^{125}$I]NDP-α-MSH at 37°C for 60 minutes.
2. A composite of receptor/[$^{125}$I]NDP-α-MSH was recovered by suction and filtration with a GFB fitter mat. Thereafter, the composite was washed with wash buffer.
3. The amount of radioactivity was measured with a scintillation counter (Top Count NXT, PerkinElmer).

<Data analysis>

1. Calculation of rate of inhibition

The rate of inhibition at each concentration of the test substance was calculated by the following equation.

$$\text{Rate of inhibition (\%)} = \{1 - (c - a)/(b - a)\} \times 100$$

a: non-specific amount of radioactivity (mean cpm value)
b: amount of radiation (mean cpm value) in the absence of test substance
c: amount of radiation (each cpm value) in the presence of test substance

2. Calculation of IC50 value

The IC50 value of the test substance was calculated by the following regression equation.

$$Y = Bottom + (Top - Bottom)/\{1 + 10^{(Log IC50 - X) \times nH}\}$$

X was the common logarithm of the concentration.
Y was fitted to a sigmoid curve having Bottom (0) and Top (100).
nH: variable Hill slope.

3. Calculation of Ki value

The Ki value of the test substance was calculated by the following equation.

$$Ki = IC50/(1 + [Ligand]/Kd)$$

[Ligand]: concentration of radioactive ligand used in assay
Kd: the historical data in Europhins Panlabs as an examination facility was seen with respect to the dissociation constant of binding of the radioactive ligand to each receptor.

4. Software

MathIQ™ (version 2.0.1.8; ID Business Solutions Ltd., UK)

Prism (version 4.03; GraphPad Software, Inc)

The results are shown in Table 2. The compound A activated MC1R at a low concentration, as compared with other MCRs, and was found to be a selective agonist against MC1R.

[Table 2]

| Human recombinant receptor | *Ki* [nmol/L] |
|---|---|
| MC1R | 2.26 |
| MC3R | 1420 |
| MC4R | 32.9 |
| MC5R | 486 |

Example 3

[Measurement of melanin production activity in mouse melanoma cell lines]

The following procedure was used to evaluate the effect of a MC1R agonist on melanin production from mouse melanoma cell lines.

(1) Cells and culture method

Mouse melanoma derived cell line B16F1 cells were used in measurement of melanin production. B16F1 cells were cultured by using Dulbecco's Modified Eagle Medium (DMEM) containing 10% fetal bovine serum (FBS) and 100 U/mL Penicillin-Streptomycin.

(2) Melanin production assay and calculation of data

B16F1 cells ($1.5 \times 10^4$ cells/well) were seeded into a 96 well plate, a culture medium containing a solution containing each concentration of the compound was added to the 96 well plate, and thereafter the cells were cultured by an incubator (set at 37°C, 5% $CO_2$) for 3 days. The melanin concentration in the medium was quantitatively determined by measuring the absorbance (405 nm) of the culture supernatant with a melanin solution as a standard solution by use of a microplate reader VERSAmax. The median value of the amount of melanin production with respect to each concentration group of NDP-$\alpha$MSH and the compound A was calculated, and the $\Delta$ value of the amount of melanin production with respect to each median value was calculated according to the following equation. One experiment was performed

in triplicate, and three independent experiments were performed .

$$\Delta \text{ Value} = \text{A - B}$$

A: median value of amount of melanin production with respect to each concentration group of each test substance
B: median value of amount of melanin production with respect to DMSO group

The maximum Δ value of NDP-αMSH was calculated by using the Δ value with respect to each concentration group of NDP-αMSH, according to non-linear regression. The maximum Δ value was defined as 100%, the Δ value with respect to each concentration group of NDP-αMSH and that of compound A were converted in terms of percentage, and thus the % value was calculated.

$$\% \text{ Value} = \text{C/D} \times 100$$

C: Δ value with respect to each concentration group of each test substance
D: maximum Δ value of NDP-αMSH calculated according to non-linear regression
EC50 (concentration at which a response corresponding to one-half of Emax with respect to NDP-αMSH was exhibited) with respect to the Compound A and NDP-αMSH ([Nle4,D-Phe7]-α-MSH) were calculated according to non-linear regression.

(3) Statistical analysis

In consideration of the correspondence of each experiment, the difference in % value from a negative control was determined by performing a two-way Dunnett's test based on each group and the number of experiments as factors. The significance level here was 5% on both sides. Such analysis was performed with SAS.
The results are illustrated in Figure 1. Both MT-7117 and NDP-αMSH induced melanin production in a concentration-dependent manner, and statistical significance was achieved at a concentration of 3 pmol/L or more.

Example 4

[Examination of effect on darkening of mouse body hair]

The following procedure was used to evaluate the effect of a MC1R agonist on darkening of body hairs of C57BL/6J-Ay/a mice.

<Test animal and test design>

Male C57BL/6JHamSlc-Ay/+ mice (Japan SLC, Inc.) were used. The test was performed with 29- or 30-days old mice, in order to use each individual whose hair cycle was at the anagen stage. The hair on the back of each of the mice was shaved with a clipper before the starting of administration, and any individual with no bad conditions such as nicks and maculae on the skin was selected, and subjected to grouping based on a group configuration shown in Table 3 by performing a simulation method so that the body weights in respective groups were uniform. Administration (also including preparation) of the test substance and evaluation of body hair were performed separately by respective different experimenters, and such evaluation was performed in a blinded manner. The starting date of administration was set to day 0, and the test substance was administered once daily for 6 days (day 0 to day 5).

<Method for preparing test substance>

Compound A: a required amount of a compound was weighed as free-form equivalent, and suspended in an aqueous 0.5% (w/v) methylcellulose (0.5%MC) solution so that an objective concentration was obtained. The compound was orally administered at a volume of 0.1 mL/10 g (body weight).
NDP-αMSH (control drug): a required amountof compound was weighed as free-form equivalent, and dissolved in Dulbecco's phosphate-buffered saline (PBS) at a concentration of 0.4 mg/mL. The drug was subcutaneously administered at a volume of 0.05 mL/10 g (body weight).

<Evaluation item>

Evaluation and sampling were performed on day 6. The mice were euthanized by cervical dislocation, thereafter subjected to hair shaving of each back with a clipper, and each individual was rated as positive (+) or negative (-) by the degree of darkening of body hair.

<Statistical analysis>

A statistical analysis was performed by SAS, and the significance level was 0.05. A 0.5% MC administration group and an NDP-αMSH group were analyzed by Fisher's exact probability test. Comparison between the 0.5% MC administration group and each compound A administration group was analyzed by Fisher's exact probability test (fixed-order test). The fixed-order test was performed by Fisher's exact probability test from a higher dose, and, if significant, performing analysis at a lower dose.

[Table 3]

| Group | Test substance | | | Number of individuals |
|---|---|---|---|---|
| | Name | Dose [mg/kg] | Route of Administration | |
| 1 | 0.5% MC | - | p.o. | 7 |
| 2 | Comoound A | 0.003 | p.o. | 7 |
| 3 | Comoound A | 0.03 | p.o. | 7 |
| 4 | Compound A | 0.3 | p.o. | 7 |
| 5 | Comoound A | 3 | p.o. | 7 |
| 6 | NDP- α MSH | 2 | s.c. | 7 |

As a result, the MC1R agonist drug significantly blackened body hair newly grown after hair shaving, at an amount of dosage of 0.3 mg/kg or 3 mg/kg (Table 4).

[Table 4]

| | | Number of mice | |
|---|---|---|---|
| Test substances | Dose (mg/kg) | Negative | Positive |
| vehicle | - | 6 | 0 |
| Comound A | 0.003 | 7 | 0 |
| Compound A | 0.03 | 7 | 0 |
| Comound A | 0.3 | 0 | 7** |
| Compound A | 3 | 0 | 7** |
| NDP- α MSH | 2 | 0 | 7## |

** indicates P < 0.01 and ## indicates P < 0.01.

Example 5

Expression of melanin-related genes of skin in clinical trial of compound A, targeted to healthy subjects

The compound A was orally administered once, to white male healthy subjects between 18 and 55 years old in a randomized double-blind clinical study. The subjects were divided to 8 subjects for placebo administration and 6 subjects for each dose of 30, 100, 300, and 600 mg of the compound A. The back skin tissue sections of the subjects were collected by biopsy punch on the day before drug administration and after one day after such administration. The intensity of mRNA expression on human skin tissues was measured with reference to kit instructions by use of GeneChip Human Gene 2.0 ST Array System (Catalog Number 902112) of Affymetrix, Inc. Melanin synthesis-related genes, tyrosinase-related protein 1 (TYRP1) and premelanosome protein (PMEL), were examined about the rate of change in gene expression and its significant difference on the next day of drug administration based on the gene expression level on the day before drug administration.

As a result, a significant increase in expression was observed with respect to TYRP1 and PMEL in each

administration group of 100, 300, and 600 mg of the compound A (Table 5).

[Table 5]

| Group | Dose [mg] | Number of cases | TYRP1 | | PMEL | |
|---|---|---|---|---|---|---|
| | | | Rate of changes (times) | P-value | Rate of changes (times) | P-value |
| Placebo | - | 8 | -1.08 | 0.371 | -1.08 | 0.427 |
| Compound A | 30 | 6 | 1.3 | 0.764 | 1.22 | 0.12 |
| Compound A | 100 | 6 | 1.37 | 0.039 | 1.77 | <0.001 |
| Compound A | 300 | 6 | 2.14 | 0.041 | 2.49 | 0.002 |
| Compound A | 600 | 6 | 2.65 | 0.001 | 3.54 | <0.001 |

Example 6

Change in skin melanin density in clinical trial of compound A, targeted to healthy subjects

The compound A was orally administered repeatedly once daily, to male healthy subjects between 18 and 55 years old for 14 days in a randomized double-blind clinical study. The subjects were divided to 12 subjects for placebo administration and 9 subjects for each dose of 30, 150, 300, and 450 mg of the compound A. The color of the skin of each of the subjects before drug administration was determined by Fitzpatrick skin classification, and the color with respect to a placebo group was scored as Type III through Type V, the color with respect to each administration group of the compound A at 30 to 300 mg was scored as Type II to Type IV, and the color with respect to an administration group at 450 mg was scored as Type V. The skin melanin density was measured by a spectrophotometric colorimeter (Model Number CM-600d) manufactured by KONICA MINOLTA, INC., with reference to the product manual.

The results were illustrated in Figure 2. An increase in melanin density of the skin was maximum on day 15, in each administration group of 150 and 300 mg of the compound A, and such an increase was kept until day 29 (Figure 2). On the other hand, no increase in melanin density after drug administration was observed because the color of the skin in an administration group of 450 mg of the compound A was scored as Fitzpatrick Type V exhibiting black color before drug administration (Figure 2).

Example 7

UV-A Irradiated Mouse Photoaging Model

<Test animals and test design>

Male albino hairless mice (strain name: Hos:HR-1, Hoshino Laboratory Animals Inc.) were used. The mice were divided into groups so that the body weight of each group was homogenous, and from the next day, the dorsal skin of the mice was irradiated with an incremental amount of ultraviolet A at the frequency of five times a week for 8 weeks to induce photoaging in the groups other than the untreated group. The UV-A irradiation schedule was based on the report by Minami et al. (J Nutrition Biochem 2009, 20, 389).

<Methods for preparation of test substances>

Compound B: The required amount was weighed as free-form equivalent, and suspended in 0.5% MC to achieve the target concentration, and orally administered at a volume of 0.1 mL/10 g (body weight) at 3 mg/kg once daily for 8 weeks.

HP-228 (reference control, melanocortin receptor-activating peptide): The required amount of free-form was weighed and dissolved in PBS to achieve the target concentration. 0.1 mL/10 g (body weight) was injected intraperitoneally at 3 mg/kg once daily for 8 weeks.

<Evaluation items>

Evaluation of skin wrinkles by visual observation (evaluation item a): The state of skin wrinkles was evaluated on a four-step scale from score 0 (fine wrinkles) to 3 (deep and coarse wrinkles) at a frequency of once a week for 8 weeks after UV-A irradiation.

Quantification of type I collagen content in the skin (evaluation item b): Mice were euthanized 8 weeks after UV-A irradiation, and their back skin was collected. Homogenates were prepared by adding acetic acid and pepsin to the collected skin. The amount of collagen in the skin homogenate, which was reduced by UV-A, was measured using the Mouse Collagen Type I ELISA Kit.

Skin histological examination (endpoint c): Skin samples were fixed in 10% neutral buffered formalin and paraffin sections were prepared. DNA damage induced by UV-A was detected by enzyme-antibody immunostaining using anti-cyclobutane pyrimidine dimer (CPD) antibody (Cosmo Bio).

<Statistical analysis>.

The skin wrinkle score was tested by Mann-Whitney's U test adjusted for multiplicity, and the significance level was set at 0.05. Significant difference test for collagen content was performed by t-test or Dunnett multiple comparison test, and the significance level was set at 0.05.

<Results

The results of evaluation item a are shown in Figure 3. The skin wrinkle scores of the compound B-group and HP-228-group were both lower or tended to be lower than those of the vehicle control group, indicating that they may have a certain anti-photoaging effect.

The results of evaluation item b are shown in Figure 4. The amount of type I collagen in the skin of the vehicle control group was degraded by UV-A irradiation and significantly decreased compared to the untreated group. The amount of type I collagen in the skin of the compound B-group and HP-228-group was significantly higher than that of the vehicle control group, indicating that compound B inhibited the degradation of skin collagen induced by UV-A.

CPD is a DNA damage marker induced by UV light, and the degree of positivity in the skin was increased by UV-A irradiation. Epidermal CPD staining in the compound B-group and HP-228-group both tended to be less positive than in the vehicle control group. These results indicate that compound B may be able to treat or prevent photodermatoses by exerting effects other than the melanin-producing effect.

Table 6

| Organ | Staining | Findings | Sex | Male | | | | | | | | | | | | | | | | | | | |
|---|---|---|---|---|---|---|---|---|---|---|---|---|---|---|---|---|---|---|---|---|---|---|---|
| | | | Group | Untreated | | | | | Vehicle | | | | | Compound B | | | | | HP-228 | | | | |
| | | | UVA-irradiation | None | | | | | UVA irradiated | | | | | | | | | | | | | | |
| | | | Number of animals | 10 | | | | | 10 | | | | | 10 | | | | | 10 | | | | |
| | | | Grading | - | ± | + | ++ | +++ | - | ± | + | ++ | +++ | - | ± | + | ++ | +++ | - | ± | + | ++ | +++ |
| Dorsal skin | CPDs (cyclobutane pyrimidine dimers immunostaining | Epidermis (cell nuclei) | | 10 | | | | | | | | 5 | 5 | | | 1 | 6 | 3 | | | 3 | 6 | 1 |
| | | Sebaceous glands(cell nuclei) | | 10 | | | | | | 1 | 9 | | | | 1 | 9 | | | | 2 | 8 | | |
| | | Cells in dermis | | 7 | 3 | | | | | | 10 | | | | | 10 | | | | | 10 | | |
| | | Hair follicles in dermis[a] (cell nuclei) | | 10 | | | | | 2 | 8 | | | | | 9 | 1 | | | 2 | 7 | 1 | | |
| | | Other tissues[b] (incl.cytoplasm in sbeceous glands) | | 3 | 7 | | | | | 9 | 1 | | | | 10 | | | | 1 | 9 | | | |

Degree of pathological findings
- : Not remarkable, ± : Minimal, + : Slight, ++ : Moderate, +++ : Severe
a) Including degenerated hair follicle (cyst)
b) Hair medulla, skin muscle, intravascular, etc.
CPD: cyclobutane pyrimidine dimers

## Claims

1. A medicament for treating or preventing a condition (excluding protoporphyria and vitiligo vulgaris) that is treatable or preventable by promotion of melanin production, the medicament comprising a compound represented by general formula [I], or a pharmaceutically acceptable salt or cocrystal thereof, as an active ingredient:

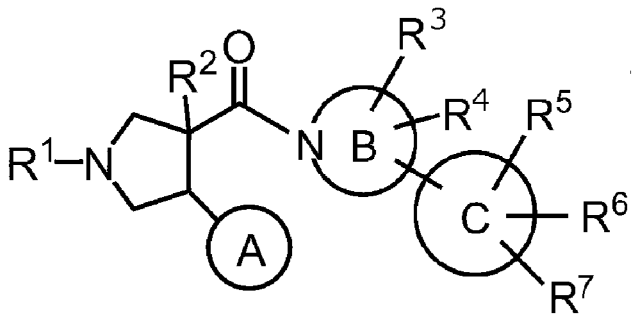

[I]

wherein ring A represents an optionally substituted aryl group or an optionally substituted heteroaryl group, wherein each substituent in the optionally substituted aryl group and the optionally substituted heteroaryl group corresponds to 1 to 3 groups independently selected from the group consisting of a halogen atom, an alkyl group, a haloalkyl group, a cycloalkyl group, an alkoxy group, a haloalkoxy group, and an alkyleneoxy group;
$R^1$ represents an optionally substituted alkyl group, an optionally substituted cycloalkyl group, an optionally substituted aliphatic heterocyclic group, an optionally substituted and optionally partially hydrogenated aryl group, an optionally substituted heteroaryl group, or a carbamoyl group optionally substituted with 1 to 2 alkyl groups,

wherein a substituent in the optionally substituted alkyl group corresponds to 1 to 3 groups independently selected from the group consisting of a halogen atom; a hydroxyl group; an oxo group; a cyano group; a cycloalkyl group; an alkoxy group; an alkanoyl group; a carbamoyl group optionally substituted with 1 to 2 alkyl groups; an aliphatic heterocyclic group; an aliphatic heterocyclic carbonyl group optionally substituted with 1 to 2 groups independently selected from the group consisting of a halogen atom, an alkyl group, a haloalkyl group, and an alkoxyalkyl group; an alkylsulfonyl group; an aliphatic heterocyclic sulfonyl group; and an alkyleneoxy group, and
each substituent in the optionally substituted cycloalkyl group, the optionally substituted aliphatic heterocyclic group, the optionally substituted and optionally partially hydrogenated aryl group, and the optionally substituted heteroaryl group corresponds to 1 to 3 groups independently selected from the group consisting of a halogen atom; a hydroxyl group; an oxo group; a cyano group; an alkyl group; a haloalkyl group; a cycloalkyl group; an alkoxy group; a hydroxyalkyl group; an alkoxyalkyl group; an alkanoyl group; a carbamoyl group optionally substituted with 1 to 2 alkyl groups; an aliphatic heterocyclic group; an aliphatic heterocyclic carbonyl group optionally substituted with 1 to 2 groups independently selected from the group consisting of a halogen atom, an alkyl group, a haloalkyl group, and an alkoxyalkyl group; an alkylsulfonyl group; an aliphatic heterocyclic sulfonyl group; and an alkyleneoxy group;

$R^2$ represents a halogen atom, an alkyl group, or an alkoxy group;
ring B represents a nitrogen-containing aliphatic heterocyclic group optionally partially including a double bond,
ring C represents an aryl group or a heteroaryl group,
$R^3$ and $R^4$ each independently represent a group selected from the group consisting of a hydrogen atom, a halogen atom, a cyano group, an alkyl group, a haloalkyl group, a cyanoalkyl group, a hydroxyalkyl group, an alkoxyalkyl group, a carboxyl group, a carbamoyl group optionally substituted with 1 to 2 alkyl groups, and an alkoxy group;
$R^5$ represents an optionally substituted alkyl group, an optionally substituted alkenyl group, an optionally substituted cycloalkyl group, an optionally substituted cycloalkenyl group, an optionally substituted aryl group, an optionally substituted heteroaryl group, an optionally substituted aliphatic heterocyclic group, an optionally substituted alkoxy group, an amino group optionally substituted with 1 to 2 alkyl groups optionally substituted with

a carboxyl group, or a carbamoyl group optionally substituted with 1 to 2 alkyl groups optionally substituted with a carboxyl group,

wherein a substituent in the optionally substituted alkyl group corresponds to 1 to 2 groups independently selected from the group consisting of a hydroxyl group; an oxo group; a cyano group; an alkoxy group; an alkanoyl group; a carboxyl group; an alkoxycarbonyl group; an aliphatic heterocyclic carbonyl group optionally substituted with a carboxyl group; a heteroaryl group optionally substituted with a hydroxyl group or a oxo group; an aliphatic heterocyclic group optionally substituted with 1 to 2 oxo groups; a carbamoyl group optionally substituted with 1 to 2 groups independently selected from the group consisting of an alkyl group (the alkyl moiety is optionally substituted with a hydroxyl group, an alkoxy group, or a carboxyl group) and a hydroxyl group; an alkylsulfonyl group; an aminosulfonyl group optionally substituted with 1 to 2 alkyl groups; an aminosulfonylaminocarbonyl group optionally substituted with 1 to 2 alkyl groups; an alkylsulfonylaminocarbonyl group; and an amino group optionally substituted with 1 to 2 groups independently selected from the group consisting of an alkyl group, an alkanoyl group, and an alkylsulfonyl group, and each substituent in the optionally substituted alkenyl group, the optionally substituted cycloalkyl group, the optionally substituted cycloalkenyl group, the optionally substituted aryl group, the optionally substituted heteroaryl group, the optionally substituted aliphatic heterocyclic group, and the optionally substituted alkoxy group corresponds to 1 to 2 groups independently selected from the group consisting of a hydroxyl group; an oxo group; a cyano group; an alkyl group optionally substituted with a carboxyl group; an alkoxy group; an alkanoyl group; a carboxyl group; an alkoxycarbonyl group; an aliphatic heterocyclic carbonyl group optionally substituted with a carboxyl group; a heteroaryl group optionally substituted with a hydroxyl group or a oxo group; an aliphatic heterocyclic group optionally substituted with 1 to 2 oxo groups; a carbamoyl group optionally substituted with 1 to 2 groups independently selected from the group consisting of an alkyl group (the alkyl moiety is optionally substituted with a hydroxyl group, an alkoxy group, or a carboxyl group) and a hydroxyl group; an alkylsulfonyl group; an aminosulfonyl group optionally substituted with 1 to 2 alkyl groups; an aminosulfonylaminocarbonyl group optionally substituted with 1 to 2 alkyl groups; an alkylsulfonylaminocarbonyl group; and an amino group optionally substituted with 1 to 2 groups independently selected from the group consisting of an alkyl group, an alkanoyl group, and an alkylsulfonyl group; and

$R^6$ and $R^7$ each independently represent a group selected from the group consisting of a hydrogen atom, a halogen atom, a cyano group, an alkyl group, a haloalkyl group, and a haloalkoxy group.

**2.** The medicament according to claim 1, wherein, in the general formula [I],

ring A is a phenyl group optionally substituted with an alkoxy group,
$R^1$ is a 5- to 6-membered monocyclic cycloalkyl group optionally substituted with a group selected from the group consisting of an alkoxy group and a cyano group,
$R^2$ is a halogen atom or an alkoxy group,
ring B is a pyrrolidinyl group, and $R^3$ is an alkoxyalkyl group and $R^4$ is a hydrogen atom or a halogen atom,
ring C is a phenyl group,
$R^5$ is a piperidinyl group substituted with a carboxyl group,
$R^6$ is a halogen atom or a haloalkyl group, and
$R^7$ is a hydrogen atom.

**3.** The medicament according to claim 1, wherein the compound represented by the general formula [I] is 1-{2-[(3S,4R)-1-{[(3R,4R)-1-cyclopentyl-3-fluoro-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid.

**4.** The medicament according to any one of claims 1 to 3, wherein the condition is photodermatoses (excluding protoporphyria).

**5.** The medicament according to claim 4, wherein the photodermatoses is photoallergic dermatitis, hydroa vacciniforme, DNA repair disorder, or porphyria (excluding protoporphyria).

**6.** The medicament according to claim 5, wherein

the photoallergic dermatitis is solar urticaria, polymorphous light eruption, chronic actinic dermatitis, photoallergic contact dermatitis, or photoallergic drug eruption, and

the DNA repair disorder is xeroderma pigmentosum, Cockayne syndrome, Bloom syndrome, Werner syndrome, Rothmund-Thomson syndrome, Trichothiodystrophy, UV-sensitive syndrome, Fanconi anemia or Ataxia telangiectasia.

**7.** The medicament according to claim 5, wherein
the porphyria is congenital erythropoietic porphyria, variegate porphyria, acute intermittent porphyria, porphyria cutanea tarda or hereditary coproporphyria.

**8.** The medicament according to any one of claims 1 to 3, wherein the condition is hypopigmentary disease (excluding vitiligo vulgaris).

**9.** The medicament according to claim 8, wherein the hypopigmentary disease is selected from
congenital hypopigmentary diseases including oculocutaneous albinism, piebaldism, nevus depigmentosus, hypomelanosis of Ito, phenylketonuria, tuberous sclerosis, dyschromatosis symmetrica hereditaria, Waardenburg syndrome, Hermansky-Pudlak syndrome, Chediak-Higashi syndrome, Griscelli syndrome, and Tietz syndrome, and acquired hypopigmentary diseases such as leukoderma Sutton, Vogt-Koyanagi-Harada syndrome, leukoderma senile, leukoderma after sea bathing, and leukoderma syphiliticum.

**10.** The medicament according to any one of claims 1 to 3, wherein the condition is an adverse effect of phototherapy.

**11.** The medicament according to any one of claims 1 to 3, wherein the condition is gray hair.

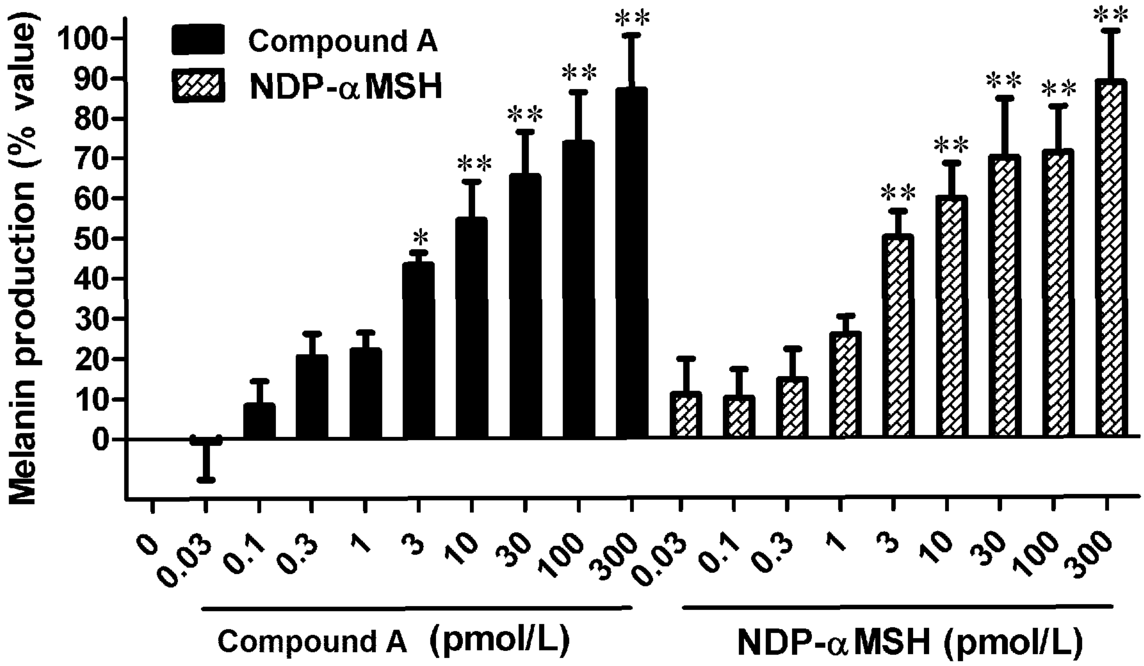

Melanin production (% value)
100
90
80
70
60
50
40
30
20
10
0
Compound A
NDP-αMSH
*
**
0
0.03
0.1
0.3
1
3
10
30
100
300
Compound A (pmol/L)
NDP-αMSH (pmol/L)


Fig. 1

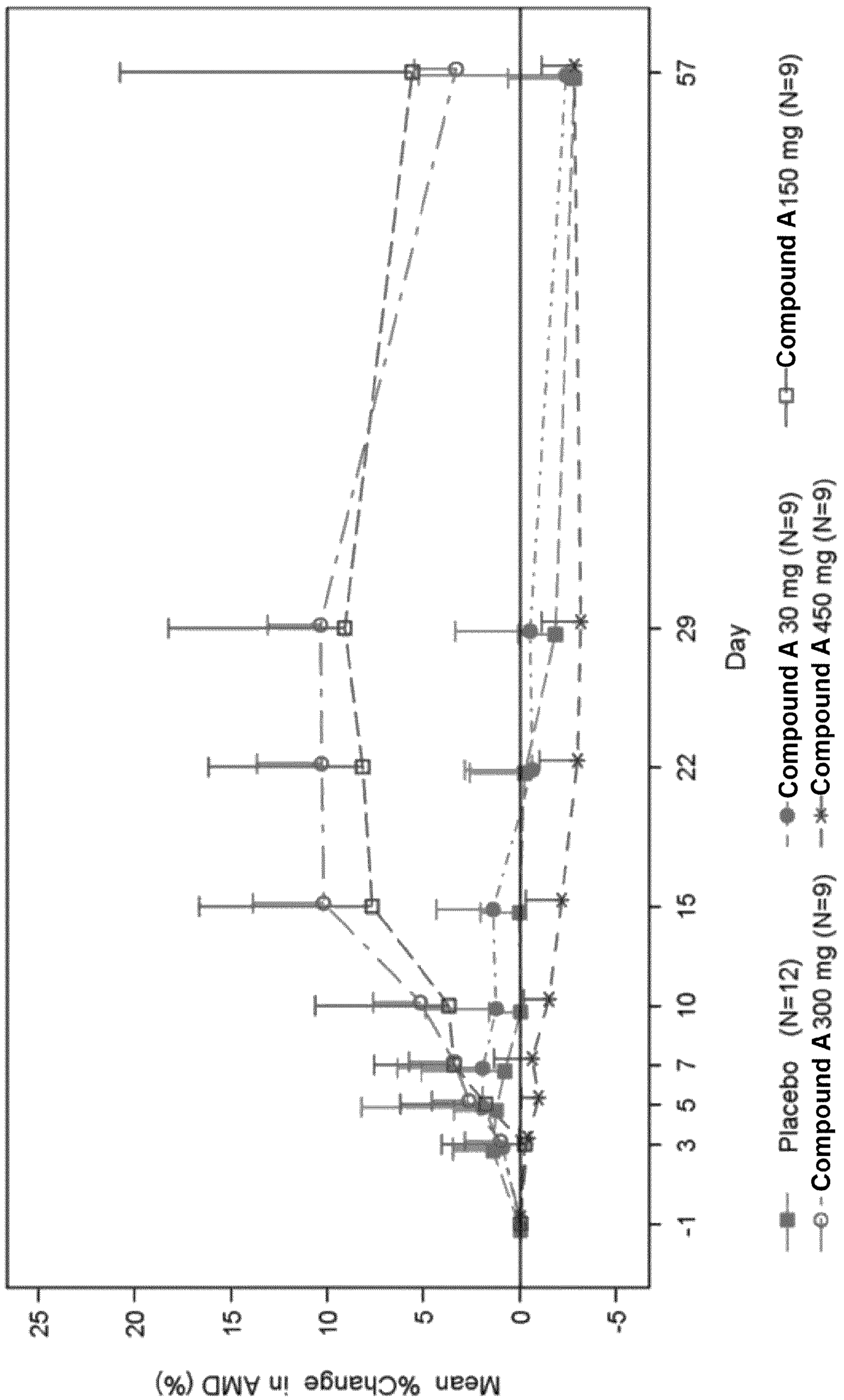
Mean %Change in AMD (%)
25
20
15
10
5
0
-5
-1
3
5
7
10
15
22
29
57
Day
Placebo (N=12)
Compound A 300 mg (N=9)
Compound A 30 mg (N=9)
Compound A 450 mg (N=9)
Compound A 150 mg (N=9)

Fig. 2

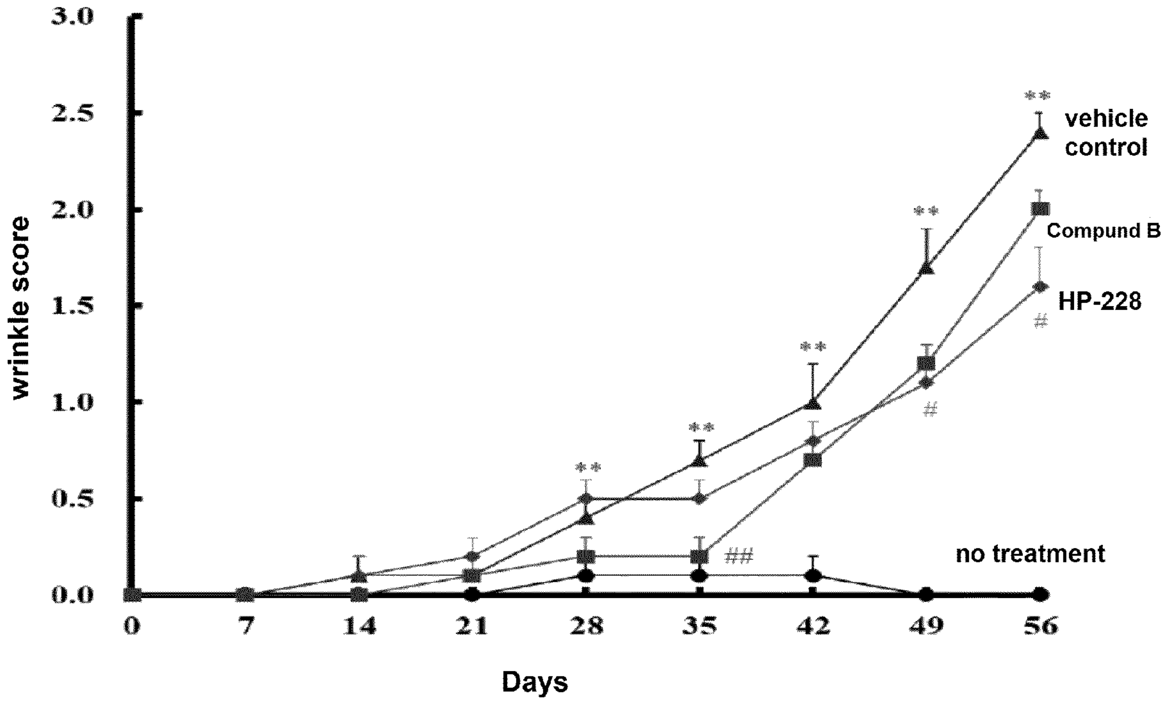
wrinkle score
3.0
2.5
2.0
1.5
1.0
0.5
0.0
0
7
14
21
28
35
42
49
56
Days
vehicle
control
Compund B
HP-228
no treatment
**
#
##

Fig. 3

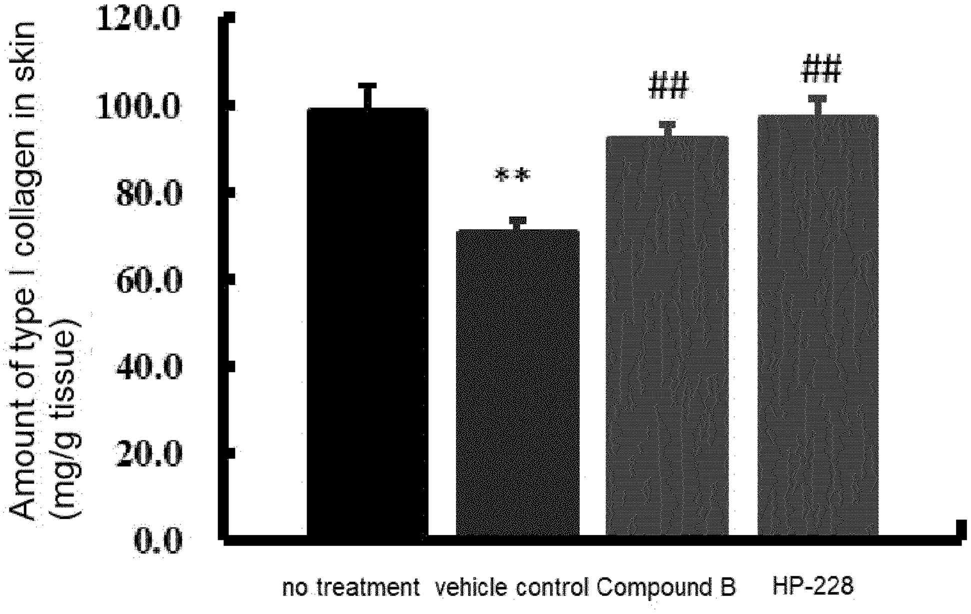
Amount of type I collagen in skin
(mg/g tissue)
120.0
100.0
80.0
60.0
40.0
20.0
0.0
no treatment
vehicle control
Compound B
HP-228
**
##

Fig. 4

## INTERNATIONAL SEARCH REPORT

International application No.
PCT/JP2021/022048

A. CLASSIFICATION OF SUBJECT MATTER
A61K 31/454(2006.01)i; A61P 17/00(2006.01)i; A61P 37/08(2006.01)i; C07D 401/14(2006.01)i
FI: A61K31/454; C07D401/14; A61P37/08; A61P17/00
According to International Patent Classification (IPC) or to both national classification and IPC

B. FIELDS SEARCHED
Minimum documentation searched (classification system followed by classification symbols)
A61K31/454; A61P17/00; A61P37/08; C07D401/14

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2017-105765 A (MITSUBISHI TANABE PHARMA CORPORATION) 15 June 2017 (2017-06-15) claims 18, 21, 22, paragraphs [0118], [0411] | 1-3, 11<br>1-11 |
| Y | JP 2014-518238 A (GALDERMA RESEARCH & DEVELOPMENT) 28 July 2014 (2014-07-28) abstract, claim 4 | 1-11 |
| Y | WO 2014/081845 A2 (UNIVERSITY OF CINCINNATI) 30 May 2014 (2014-05-30) examples 5, 6, fig. 7 | 1-11 |
| Y | MINDER, Elisabeth I. et al., "Pharmacokinetics and Pharmacodynamics of Afamelanotide and its Clinical Use in Treating Dermatologic Disorders", Clinical Pharmacokinetics, 2017, vol. 56, no. 8, pp. 815-823, ISSN. 0312-5963 abstract | 1-11 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 July 2021 (20.07.2021) | 03 August 2021 (03.08.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/022048

| Patent Documents referred in the Report | Publication Date | Patent Family | Publication Date |
|---|---|---|---|
| JP 2017-105765 A | 15 Jun. 2017 | (Family: none) | |
| JP 2014-518238 A | 28 Jul. 2014 | US 2014/0228342 A1<br>abstract, claim 4<br>US 2016/0237065 A1<br>WO 2013/001030 A1<br>EP 2726478 A1<br>FR 2981933 A<br>AU 2012277838 A<br>CA 2840606 A<br>KR 10-2014-0050019 A<br>CN 103827106 A<br>MX 2014000170 A<br>AR 86803 A<br>RU 2014102966 A<br>ES 2620476 T<br>IN 711CHN2014 A<br>BR 112013033861 A | |
| WO 2014/081845 A2 | 30 May 2014 | US 2015/0299251 A1<br>examples 5, 6, fig. 7<br>US 2015/0297667 A1<br>US 2018/0066016 A1<br>EP 2922565 A1<br>EP 2925340 A1<br>AU 2013348043 A<br>CA 2892134 A<br>MX 2015006342 A<br>AU 2013348044 A<br>CA 2892144 A<br>MX 2015006333 A<br>AU 2018204153 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014160015 A
- WO 2018142318 A
- WO O2008025094 A
- WO 2009103816 A
- WO 2015067503 A
- WO 2008025094 A
- WO 2015182723 A
- WO 2020138481 A


### Non-patent literature cited in the description


- **VINOD KUMAR SHARMA et al.** Photodermatoses in Pigmented Skin. *Photochem Photobiol Sci,* January 2013, vol. 12 (1), 65-77
- **MORIWAKI et al.** *J Dermatol,* 2017, vol. 44, 1087
- **KUNISADA et al.** *J Invest Dermatol,* 2017, vol. 137, 1975
- **NAKAZAWA et al.** *Cell,* 2020, vol. 180, 1228